# EUROPEAN PATENT APPLICATION

(11) **EP 2 287 202 A1**
(43) Date of publication of application: **23.02.2011**
(21) Application number: 09167495.2
(22) Date of filing: 07.08.2009
(51) Int. Cl.: C07K 16/44

(54) **Anti-sialic acid antibody molecules**

(71) Applicant: Dublin City University, Dublin 9 (IE)
(72) Inventor: The designation of the inventor has not yet been filed
(74) Representative: Purdy, Hugh Barry

(57) **Abstract**

A method of generating and isolating recombinant high affinity anti-sialic acid antibody molecules comprises the steps of immunising an avian host with an immunogen comprising a conjugate of sialic acid and a carrier protein to generate an anti-sialic acid polyclonal serum, isolating a sample of RNA from the immunised avian host, using phage display in the generation and screening of a library of recombinant avian antibody molecules from the RNA sample, and isolating recombinant high affinity anti-sialic acid antibody molecules. The antibody molecule is selected from the group consisting of: whole antibodies; scFv fragments; and Fab fragments, and the avian host is *Gallus domesticus.* A recombinant avian antibody fragment having high binding affinity to sialic acid and obtainable by the method of the invention, and an anti-sialic acid polyclonal serum obtainable by immunising an avian host with a conjugate of sialic acid and carrier protein, are also described.

## Description

### Introduction

The invention relates to a method of generating a library of recombinant anti-sialic acid antibody fragments, and anti-sialic acid antibody fragments obtainable by the method of the invention.

### Background to the Invention

Sialic acids are acidic monosaccharides that reside as terminal monosaccharides on *N-* and *O-*linked glycans. They are actively involved in a plethora of biological phenomena, ranging from cell-cell adhesion and recognition, intracellular signaling events, pathogen attack, viral infection, inflammatory disease and cancer. These moieties belong to a family of α-keto acids that are structurally characterised by the presence of a nine-carbon backbone. The three major forms of sialic acid are Neu5Ac, Neu5Gc and 2-keto-3-deoxy-D-glycero-D-galacto-nononic acid (KDN). Neu5Ac is the most ubiquitous sialic acid, in eukaryotic cells it is α(2,3) or α(2,6) linked to galactose and α(2,6)-linked to *N*-acetyl-galactosamine (GalNAc). Sialic acid moieties may also interact with each other, predominantly through α(2,8) and, to a lesser extent, α(2,9) linkages to form polysialic acid (PSA, or colominic acid). In addition to these basic forms, more than 50 distinct sialic acid structures have been identified in nature, arising from acetylation, methylation, lactylation, sulfation, and phosphorylation of the C-4, C-5, C-7, C-8, or C-9 hydroxyl groups. ^{1,2,3,4,5 and 6} A number of sensitive methods have been published that either measure total sialic acid content or different sialic acid types. However, these methods are time consuming, costly, laborious and may require sophisticated instrumentation as well as a high degree of operator knowledge for implementation. Hence, these assay formats are not ideally suited for routine use with complex sample types. The colorimetric determination of sialic acid, for example uses orcinol, resorcinol, periodic and thiobarbituric acid. These reagents are quite toxic and a prerequisite for performing this assay is the availability of a purified sample as lipid or 2-deoxyribose contamination generates erroneous results.

Lectin-based assays are available for certain types of sialic acid (*Sambucus nigra* and *Maackia amurensis*-derived lectins for the detection of α(2,3) and α(2,6)-linked sialic acid, respectively). However, the use of some lectins for assay development is hindered by their low sensitivity and poor specificity. The detection of sialic acid in the context of a sialoglycoprotein or as a free moiety may be facilitated by a panel of sensitive analytical techniques. These include high-performance liquid chromatography (HPLC), gas-chromatography combined with mass spectrometry (GC-MS), nuclear magnetic resonance spectrometry (NMR) and capillary electrophoresis (CE). However, these methods require significant sample preparation, specialised equipment, purification of the target protein and often require lengthy and complex data analysis for monitoring sialylation.

### Statement of Invention

According to the invention, there is provided a method of generating a library of recombinant high affinity anti-sialic acid antibody molecules, the method comprising the steps of:
- immunising an avian host with an immunogen comprising a conjugate of sialic acid and a carrier protein to generate an anti-sialic acid polyclonal serum;
- isolating a sample of RNA from the immunised avian host; and
- using phage display in the generation and screening of a library of recombinant avian antibody molecules from the RNA sample.

In another aspect, the invention relates to a method of generating a recombinant avian antibody molecule having high-affinity binding to sialic acid, the method including the steps of:
- immunising an avian host with an immunogen comprising a conjugate of sialic acid and a carrier protein to generate an anti-sialic acid polyclonal serum;
- isolating a sample of RNA from the immunised avian host;
- using phage display for the generation and screening of a library of recombinant avian antibody molecules from the RNA sample; and
- isolating a clone having high-affinity binding to sialic acid.

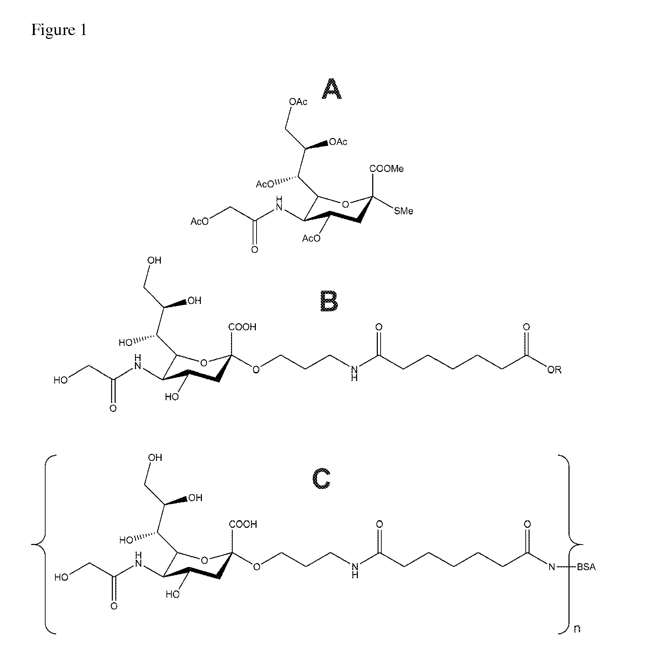

The carrier protein typically comprises any serum albumin protein, generally bovine serum albumin (BSA) or human serum albumin (HSA), conjugated to a sialic acid. Suitably, then sialic acid is Neu5Gc or Neu5Ac, ideally Neu5Gc. Suitably, the sialic acid is conjugated to the carrier protein by a linker. Preferably, the linker is a hydrocarbon chain having at least five, six, seven, eight, nine or ten carbon atoms. Ideally, a plurality of sialic acid molecules is conjugated to a single carrier protein, typically via linker molecules. In one embodiment, the conjugate has a formula shown in Fig. 1C, in which XSA is a serum albumin protein, suitably selected from the group consisting of: HSA; and BSA.

In this specification, the term "sialic acid" should be understood as meaning Neu5Gc or Neu5Ac, or both. Thus, the anti-sialic acid antibody fragments generated according to the methods of the invention will have specific binding affinity for Neu5Gc, Neu5Ac, or ideally both Neu5Gc and Neu5Ac.

The terms "high binding affinity to sialic acid" should be understood to mean a binding affinity to sialic acid (ideally both Neu5Gc and Neu5Ac) of at least micromolar affinity, ideally at least nanomolar affinity, as determined by Biacore kinetic analysis. Likewise, the term "high affinity" should be understood to mean a binding affinity to sialic acid (ideally both Neu5Gc and Neu5Ac) of at least micromolar affinity, ideally at least nanomolar affinity, as determined by Biacore kinetic analysis.

The antibody molecules generated according to the methods of the invention are selected from the group consisting of: whole antibodies, and antibody fragments, for example scFv fragments or Fab fragments. In one embodiment of the invention, the RNA sample derived from the avian host is obtained from a biological sample selected from the group consisting of: bone marrow; and spleen. Ideally, the avian RNA sample is obtained from both of bone marrow and spleen cells.

The methods of the invention provide a library of clones, each clone comprising a phage displaying a recombinant avian anti-sialic acid antibody molecule. Clones expressing high affinity antibody molecules (for example, antibody fragments that bind sialic acid, especially Neu5Gc, Neu5Ac, or ideally both) are generally isolated by means of biopanning against decreasing concentrations of sialic acid. Ideally, sialic acid is immobilised during the biopanning process. Antibody fragments are produced by means of amplification in a suitable producer cell, for example *Escherichia coli.*

In a preferred embodiment of the invention, the avian host is *Gallus domesticus.* However, other suitable avian hosts will be apparent to the skilled person, especially other related species that do not contain Neu5Gc as part of its glycome.

The library construction process involves the use of variable heavy chain (V_{H}) primers, variable light chain (V_{L}) primers, and overlap extension primers. Suitably, the V_{H} primers are the primers shown in SEQUENCE ID NO'S 1 and 2. Suitably, the V_{L} primers are the primers shown in SEQUENCE ID NO'S 3 and 4. Suitably, the overlap extension primers are the primers shown in SEQUENCE ID NO'S 5 and 6.

The invention also relates to a phage display library obtainable by the method of the invention (in which all or most of the phage, display a high affinity recombinant avian anti-sialic acid antibody molecule).

The invention also relates to a library of recombinant avian anti-sialic acid antibody molecules obtainable by means of the method of the invention.

The invention also relates to a recombinant avian antibody fragment having high binding affinity to sialic acid, ideally to both Neu5Gc and Neu5Ac.

The invention also relates to an anti-sialic acid polyclonal serum obtainable by immunising an avian host with a conjugate of sialic acid and carrier protein, ideally a conjugate of Neu5Gc and BSA or HSA.

The invention also relates to a recombinant avian anti-sialic acid antibody molecule.

In this specification, the term "anti-sialic acid" should be understood as meaning having a binding affinity to Neu5Gc or Neu5Ac (or both) of at least micromolar, and ideally at least nanomolar, affinity, as determined by Biacore kinetics. Ideally, the antibody is both anti-Neu5Gc and anti-Neu5Ac.

Ideally, the antibody molecule is an antibody fragment, typically a Fab or scFv fragment.

In one embodiment, the antibody has a CDRL2 region of amino acid sequence XNTNRPS (SEQUENCE ID NO: 7), ideally DNTNRPS (SEQUENCE ID NO: 8). These sequences are consensus sequences from the CDRL2 regions of clones AE8, AG9, CD3, and CC11.

In one embodiment, the antibody has a CDRL3 region of amino acid sequence GXY/FDXSXXXXXX (SEQUENCE ID NO: 9), preferably GXXDXSAXXXXI (SEQUENCE ID NO: 10), and ideally GSYDRSAGYVGI (SEQUENCE ID NO: 11). These sequences are consensus sequences from the CDRL3 regions of clones AE8, AG9, CD3, and CC11.

In one embodiment, the antibody has a CDRL1 region of amino acid sequence XXXXXXXYG (SEQUENCE ID NO: 12), suitably SGGXXSXYG (SEQUENCE ID NO: 13), and ideally SGGGGSYYG (SEQUENCE ID NO: 14). These sequences are consensus sequences from the CDRL1 regions of clones AE8, AG9, CD3, and CC11.

In one embodiment, the antibody has a CDRH1 region of amino acid sequence GFXFXXXXMX (SEQUENCE ID NO: 15), suitably GFTFXSXXMX (SEQUENCE ID NO: 16), and ideally GFTFDSYAMY (SEQUENCE ID NO: 17). These sequences are consensus sequences from the CDRH1 regions of clones AE8, AG9, CD3, and CC11.

In one embodiment, the antibody has a CDRH2 region of amino acid sequence IXXXGXXTXXGAAV (SEQUENCE ID NO: 18), suitably INRFGS/NSTGHGAAV (SEQUENCE ID NO: 19). These sequences are consensus sequences from the CDRH2 regions of clones AE8, AG9, CD3, and CC11.

In one embodiment, the antibody has a CDRH3 region of amino acid sequence SXXGXXXXXXXXXXXXIDA (SEQUENCE ID NO: 20), suitably SVHGS/HCASGT/YWCSP/AASIDA (SEQUENCE ID NO: 21). These sequences are consensus sequences from the CDRH3 regions of clones AE8, AG9, CD3, and CC11.

In the sequence listings above, "X" denotes either any amino acid, or no amino acid. Further, "X/D" denotes that the residue at that position is X (as defined above) or D.

In one preferred embodiment of the invention, the antibody comprises a polypeptide having a CDRL1 region according to SEQUENCE ID NO: 12, 13 or 14, a CDRL2 region according to SEQUENCE ID NO: 7 or 8, and a CDRL3 region according to SEQUENCE ID NO'S: 9, 10 or 11, or a functional variant of the polypeptide.

In one preferred embodiment of the invention, the antibody comprises a polypeptide having a CDRH1 region according to SEQUENCE ID NO: 15, 16 OR 17, a CDRH2 region according to SEQUENCE ID NO: 18 or 19, and a CDRH3 region according to SEQUENCE ID NO'S: 20 or 21, or a functional variant of the polypeptide.

In a particularly preferred embodiment of the invention, the antibody comprises a polypeptide having a CDRL1 region according to SEQUENCE ID NO: 12, 13 or 14, a CDRL2 region according to SEQUENCE ID NO: 7 or 8, a CDRL3 region according to SEQUENCE ID NO'S: 9, 10 or 11, a CDRH1 region according to SEQUENCE ID NO: 15, 16 or 17, a CDRH2 region according to SEQUENCE ID NO: 18 or 19, and a CDRH3 region according to SEQUENCE ID NO'S: 20 or 21, or a functional variant of the polypeptide. Ideally, the antibody is an antibody fragment, typically an scFv antibody.

The invention also provides an antibody molecule comprising a heavy chain variable region and a light chain variable region, the light chain variable region comprising a CDRL1 region according to SEQUENCE ID NO: 12, 13 or 14, a CDRL2 region according to SEQUENCE ID NO: 7 or 8, a CDRL3 region according to SEQUENCE ID NO'S: 9, 10 or 11, the heavy chain variable region comprising a CDRH1 region according to SEQUENCE ID NO: 15, 16 or 17, a CDRH2 region according to SEQUENCE ID NO: 18 or 19, and a CDRH3 region according to SEQUENCE ID NO'S: 20 or 21.

The invention also provides an antibody molecule comprising a heavy chain variable region and a light chain variable region, the light chain variable region comprising a CDRL1 region according to SEQUENCE ID NO: 14, a CDRL2 region according to SEQUENCE ID NO: 8, a CDRL3 region according to SEQUENCE ID NO'S: 11, the heavy chain variable region comprising a CDRH1 region according to SEQUENCE ID NO: 17, a CDRH2 region according to SEQUENCE ID NO: 19, and a CDRH3 region according to SEQUENCE ID NO'S: 21.

The invention also provides an antibody molecule comprising a heavy chain variable region and a light chain variable region, the light chain variable region comprising a sequence according to SEQUENCE ID NO: 22, or a functional variant thereof, and the heavy chain variable region comprising a sequence according to SEQUENCE ID NO: 23, or a functional variant thereof.

The invention also provides an antibody molecule comprising a heavy chain variable region and a light chain variable region, the light chain variable region comprising a sequence according to SEQUENCE ID NO: 24, or a functional variant thereof, and the heavy chain variable region comprising a sequence according to SEQUENCE ID NO: 25, or a functional variant thereof.

The invention also provides an antibody molecule comprising a heavy chain variable region and a light chain variable region, the light chain variable region comprising a sequence according to SEQUENCE ID NO: 26, or a functional variant thereof, and the heavy chain variable region comprising a sequence according to SEQUENCE ID NO: 27, or a functional variant thereof.

The invention also provides an antibody molecule comprising a heavy chain variable region and a light chain variable region, the light chain variable region comprising a sequence according to SEQUENCE ID NO: 28, or a functional variant thereof, and the heavy chain variable region comprising a sequence according to SEQUENCE ID NO: 29, or a functional variant thereof.

The invention also relates to an antibody molecule comprising a sequence selected from the group consisting of: SEQUENCE ID NO: 30; 31; 32; and 33.

Suitably, the antibody molecule is an scFv antibody fragment in which the light chain variable region and heavy chain variable region are connected in series in a single molecule, usually by means of a linker. However, the antibody molecule may also be in the form of a Fab antibody fragment.

In this specification, the term "functional variant" should be understood as meaning a variant of the antibody-related polypeptide having at least 80%, 90%, and ideally at least 95%, 96%, 97%, 98% or 99% sequence identity (homology) with the listed polypeptides and which have a binding affinity for sialic acid that is not less than 80% that of the listed polypeptides, and ideally not less than 90% that of the listed polypeptides. The term should be taken to include antibody molecules that are altered in respect of one or more amino acid residues. Preferably such alterations involve the insertion, addition, deletion and/or substitution of 5 or fewer amino acids, more preferably of 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. In this context, sequence homology comprises both sequence identity and similarity, i.e. an antibody molecule that shares 70% amino acid homology with a listed is one in which any 70% of aligned residues are either identical to, or conservative substitutions of, the corresponding residues in the listed antibody molecule.

The term "variant" is also intended to include chemical derivatives of the listed antibody molecules, i.e. where one or more residues of the antibody molecule is chemically derivatised by reaction of a functional side group. Also included within the term variant are antibody molecules in which naturally occurring amino acid residues are replaced with amino acid analogues.

Antibody molecules (including variants and fragments thereof) of and for use in the invention may be generated wholly or partly by phage display, chemical synthesis or by expression from nucleic acid. The proteins and peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984).

The invention also relates to a conjugate of sialic acid to a carrier protein. Typically, the sialic acid is Neu5Gc or Neu5Ac. Suitably, the carrier protein is a serum albumin, typically HSA or BSA. Ideally, the sialic acid is conjugated to the carrier protein by means of a linker. Suitably, the linker is a hydrocarbon chain having at least five, six, seven, eight, nine or ten carbon atoms in the backbone, and optionally comprises a peptide bond intermediate the ends of the chain. Ideally, the conjugate has the formula shown in Fig. 1C, in which XSA is a serum albumin protein, suitably selected from the group consisting of: HSA; and BSA.

The invention also relates to the use of a conjugate of the invention as an immunogen in the generation of anti-sialic acid antibodies.

The invention also relates to a method of identifying and/or quantifying the presence of a sialic acid in a sample comprising the steps of binding an antibody molecule of the invention with the sample, and then detecting an immuno-specific reaction between the antibody molecule and sialic acid.

The invention also relates to an ELISA kit suitable for identifying and/or quantifying the presence of a sialic acid in a sample, the kit comprising an antibody molecule of the invention immobilised to a support, and optionally one or more diagnostic reagents capable of detecting and/or quantifying any immunospecific reaction between the antibody molecule and sialic acid.

The invention also relates to the use of an antibody molecule of the invention as a targeting vector for targeting a molecule to a specific locus in a sample. The molecule to be targeted may be, for example, a pharmaceutically active agent such as an anti-cancer drug or a drug suitable for treatment of a neurological disorder, or an imaging molecule such as a dye. The sample may be a cell, tissue, an organ or a body. Thus, the use is intended for both *in-vitro, in-vivo,* and *ex-vivo* applications, and for the purpose of therapy, diagnosis, and research.

### Brief description of figures:

**Figure 1****:** The synthesis of the BSA and HSA-Neu5Gc containing conjugates. A batch of a donor derivative (compound A) of Neu5Gc was synthesised from the free sugar. Compound A was further derivatised to form a new derivative with an activated ester at the end of a long chain (compound B). Compound B was reacted with the side chain amines of the protein's (HSABSA) lysine residues, forming amide bonds with the sugar derivative (compound C). The pre-activated form of compound B was checked by NMR to ensure structural correctness and by TLC to determine purity level. The final product, compound C, was checked by MALDI-MS to give an accurate figure for the degree of sugar substitution to the protein.

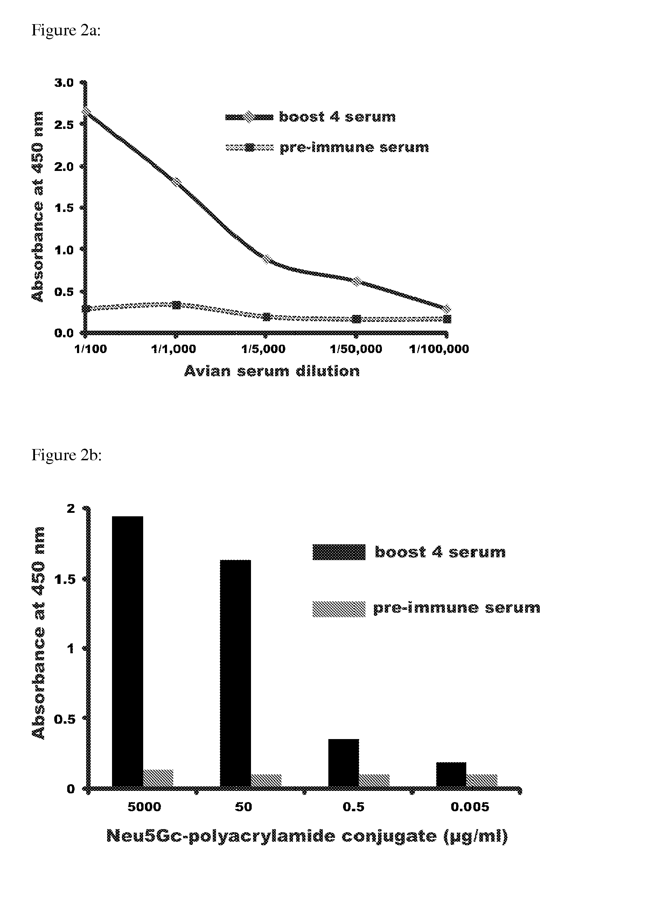

**Figure 2a****:** Titre of an avian antiserum response to a Neu5Gc-BSA conjugate.
**Figure 2b****:** Titre of an avian antiserum response to a Neu5Gc-polyacrylamide conjugate.

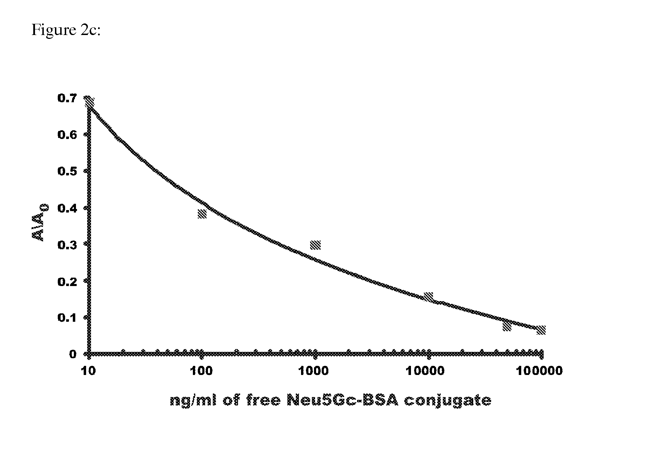

**Figure 2c****:** Inhibition ELISA of the avian antiserum with free Neu5Gc-BSA conjugate. The serum-based antibodies were able to compete between free and immobilised Neu5Gc-BSA, verifying that the response was sialic acid-specific.

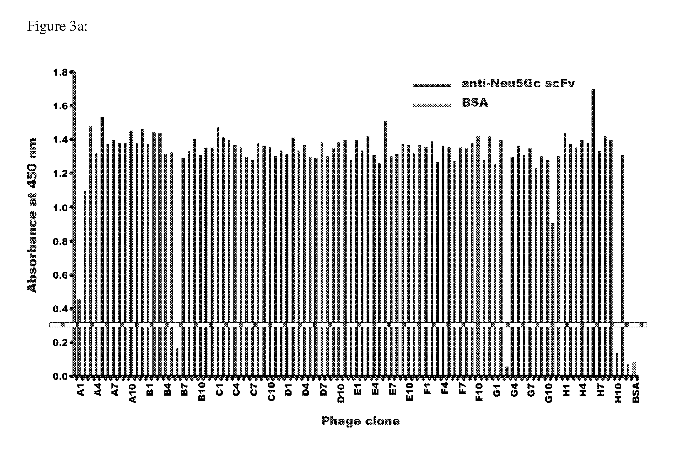

**Figure 3a****:** Soluble monoclonal phage ELISA of competitively-eluted phage clones from rounds three and four of biopanning. The ELISA threshold is marked with a horizontal line. The BSA bar represents the total average binding of all clones to BSA. A selection of positive clones is illustrated.

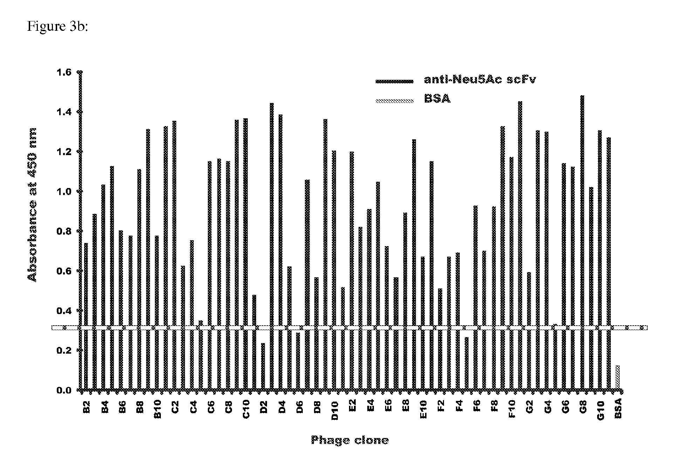

**Figure 3b****:** Soluble monoclonal phage ELISA of trypsin-eluted phage clones from rounds three and four of biopanning. The ELISA threshold is marked with a horizontal line. The BSA bar represents the total average binding of all clones to BSA.

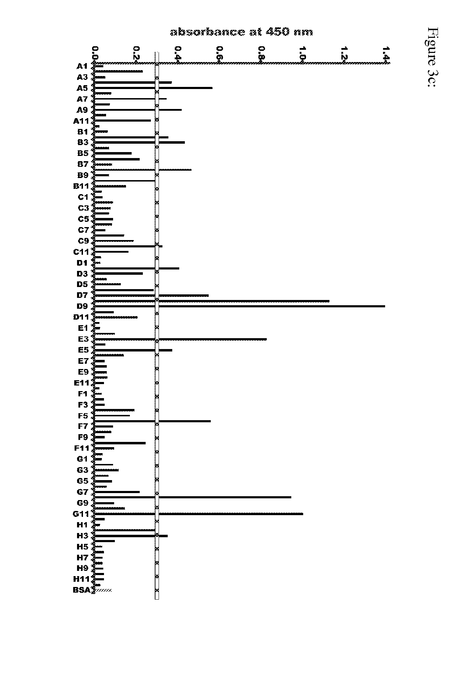

**Figure 3c****:** Soluble anti-sialic acid clones that recognise Neu5Gc in the form of both Neu5Gc-BSA and Neu5Gc-PAA. The ELISA threshold is marked with a horizontal line. The BSA bar represents the total average binding of all clones to BSA. A small selection of positive clones is shown.

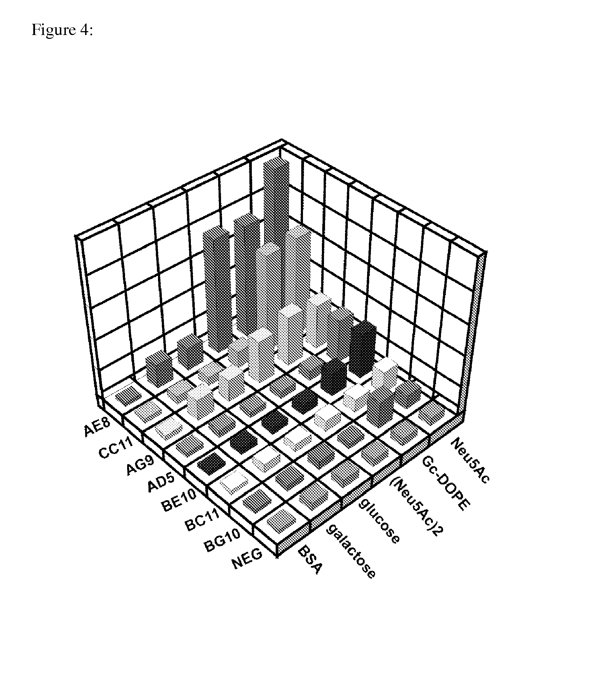

**Figure 4****:** A cross reaction study of soluble anti-Neu5Gc scFvs to different monosaccharides and sialic acids. Clone AE8 can recognise sialic acid in the context of Neu5Ac, Neu5Gc and polysialic acid (PSA). Moreover, no significant binding to galactose or glucose was observed.

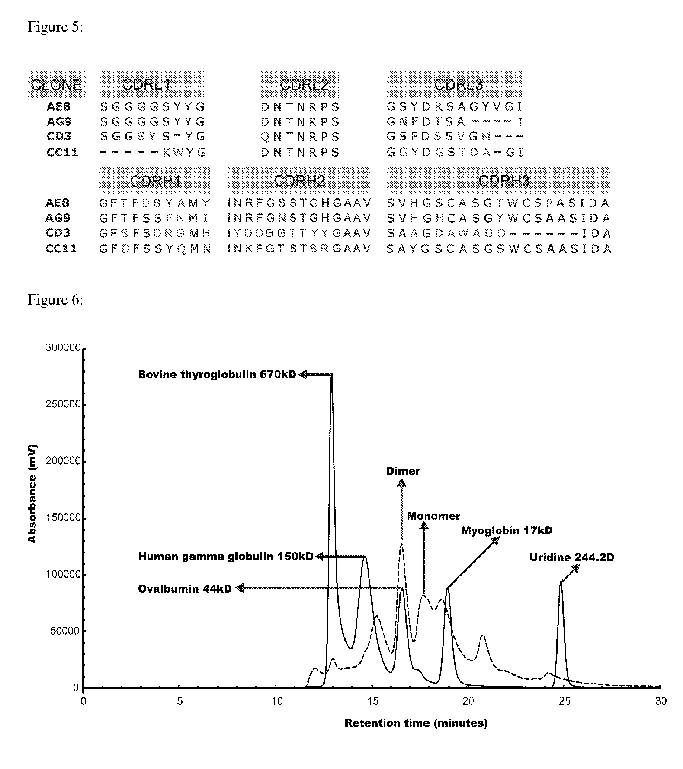

**Figure 5****:** Comparative sequence analysis of five different sialic acid-binding clones. All clones were sequenced in triplicate. All five scFv genes are different, clone CD3 shows the greatest differences in both the heavy and light chain regions. AE8 and AG9 show only subtle differences in CDRL3, CDRH1, CDRH2 and CDRH3 regions.
**Figure 6****:** SEC-HPLC analysis of IMAC-purified AE8. The protein standards are represented by the black solid line. A neat sample of purified AE8 (represented by the black dotted line) in 1x PBS (pH 7.2) was applied to the HPLC column. A Phenomenex 3000 SEC column was used with 1x PBS (pH 7.2) mobile phase at a flow rate of 0.5ml/minute and monitored by UV absorbance at 280nm. The scFv dimer peak was observed at 16.6 minutes and a smaller monomer peak eluted at 17.7 minutes.

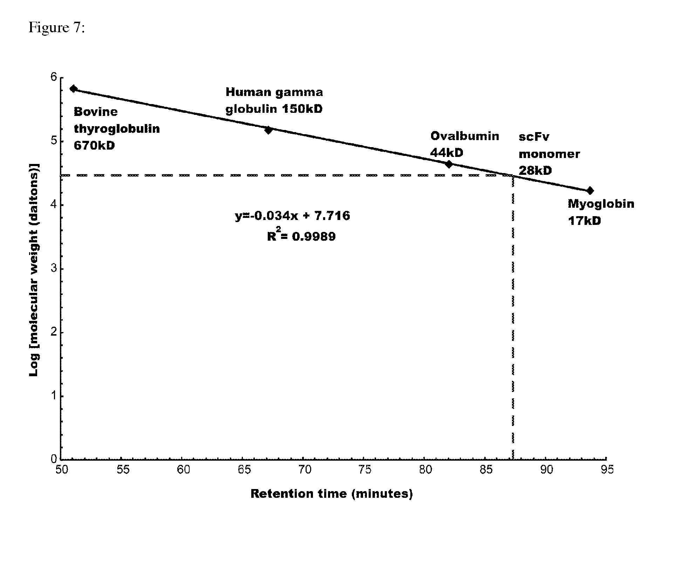

**Figure 7****:** FPLC analysis of the IMAC-purified anti-sialic acid scFv (AE8). 100µl of the AE8 sample was applied to a HiLoad™ 16/60 Superdex™ 200 Prep-grade FPLC column using filtered and degassed 1x PBS (pH 7.4) at a flow rate of Iml/min. The retention time of the calibration standards indicated are Bovine Thyroglobulin, 670 kDa, 51.09 min; Human gamma globulin, 150 kDa, 67.15 min; Ovalbumin, 44 kDa, 82.03 min and Myoglobin, 17 kDa, 93.65 min. The coefficient of determination for the standard curve (y=-0.034x + 7.716) was R² = 0.9989. The retention time of the monomeric AE8 scFv fraction was 87.36 min and this yielded an estimated molecular weight of 28kDa.

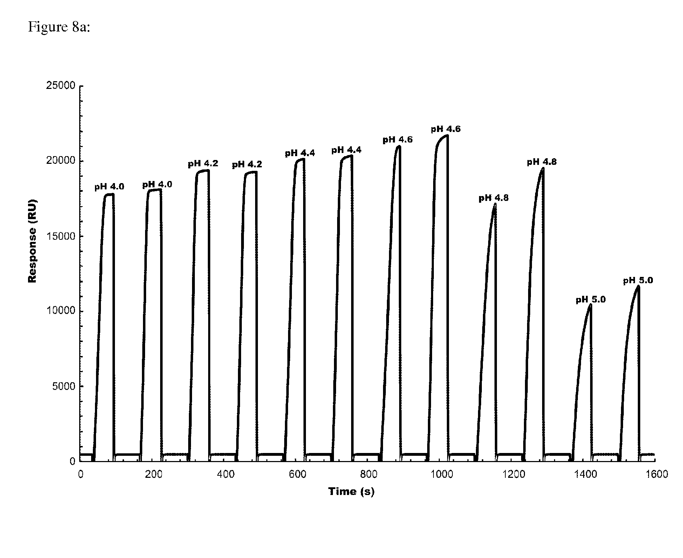

**Figure 8a****:** Neutravidin pre-concentration analyses. 50µg/ml solutions of neutravidin were prepared in 10mM sodium acetate buffers and adjusted with 10% (v/v) acetic acid to pH values 4.0, 4.2, 4.4, 4.6, 4.8, and 5.0. A 10mM sodium acetate buffer at pH 4.6 was chosen as the immobilisation buffer for neutravidin on the CM5 surface.

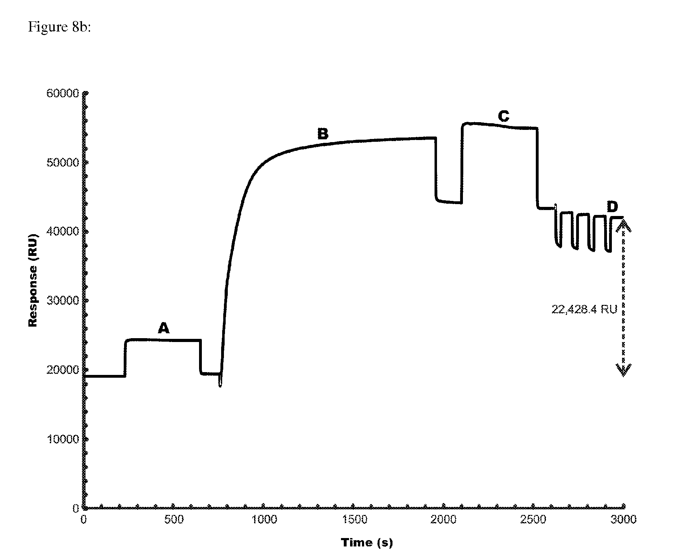

**Figure 8b****:** Immobilisation of 50µg/ml neutravidin onto the CM5 sensor chip surface. (A) EDC/NHS activation, (B) binding of neutravidin, (C) capping of unreacted groups and (D), regeneration pulses of 5mM NaOH. A final level of 22,428.4 RUs of covalently attached neutravidin was achieved.

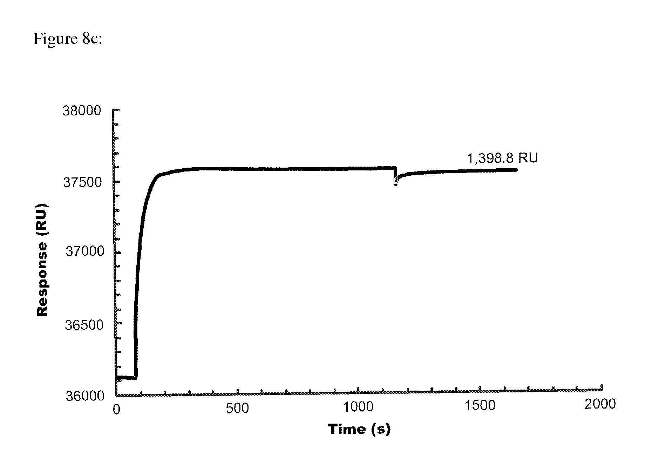

**Figure 8c****:** The response profile of biotinylated-Neu5Gc-PAA binding to a CM5 chip with immobilised neutravidin. A 100µg/ml solution of biotinylated-Neu5Gc-PAA in HBS was passed over the chip surface at 10µl/min for 20 minutes. A final level of 1,398.8 RU of captured biotinylated-Neu5Gc-PAA was achieved.

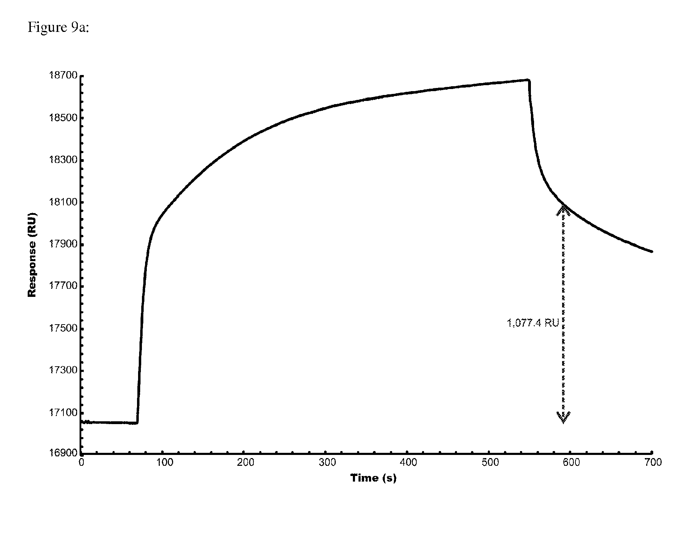

**Figure 9a****:** A reference subtracted sensorgram depicting the binding of the AE8 recombinant anti-sialic scFv to Neu5Gc. The AE8 protein was purified by IMAC and a 1 in 100 dilution in HBS was passed over flow cells 1 and 2 of the Neu5Gc senor chip at 10µl/min for 7 minutes.

The net binding response achieved by AE8 was 1,077.4 RU. The reference subtracted control surface (flow cell 1) consisted of immobilised neutravidin with no biotinylated Neu5Gc-PAA.

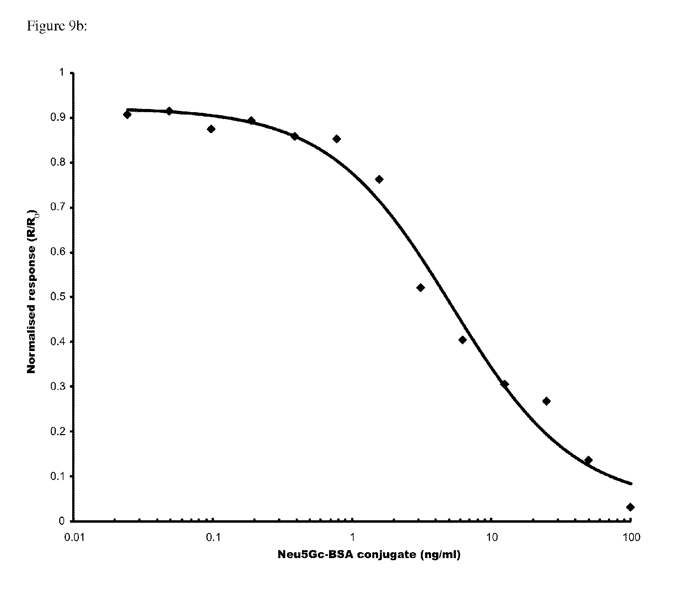

**Figure 9b****:** A Biacore inhibition curve of the anti-sialic binding scFv (AE8). The R/Rₒ was calculated by dividing the RU response obtained at different Neu5Gc-BSA conjugate concentrations (1000ng/ml, 500ng/ml, 250ng/ml, 125ng/ml, 62.5ng/ml and 31.25ng/ml) by the RU response obtained from the AE8 sample with no Neu5Gc-BSA conjugate. A 4-parameter equation was fitted to the data set using BIAevaluation 4.1 software. Each point in the curve is the mean of three replicate measurements.

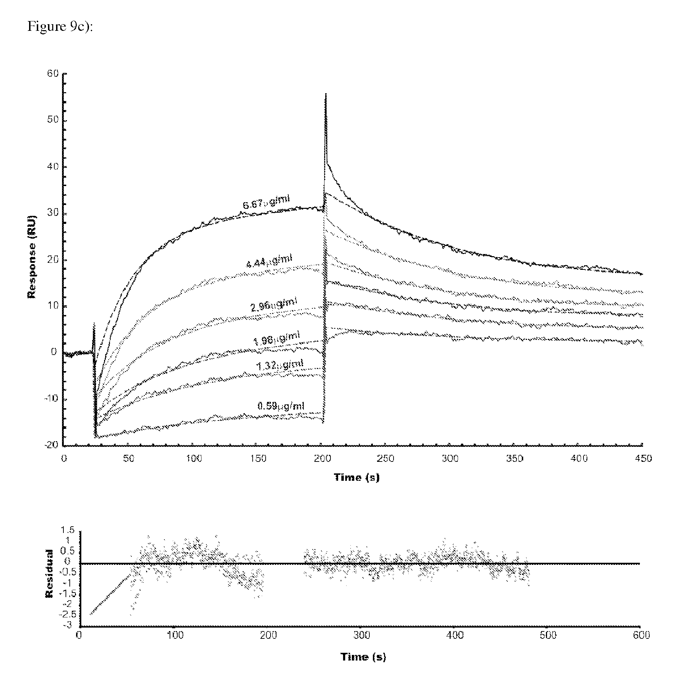

**Figure 9c****:** Curve fitting of monomeric AE8 scFv with BIAevaluation 4.1 using a 1:1 binding model. The fitted curves for each monomeric scFv concentration are represented by the dashed line, whereas the solid lines represent the actual RU change for each sample. The apparent K_{D} was estimated to be 57x 10⁻⁹ M. The residual plot is a measure of the 'goodness of fit'.

### Detailed Description of the Invention

### 1.0. New strategies for immunisation to generate anti-sialic acid antibodies

The generation of anti-carbohydrate antibodies is a notoriously difficult task. Carbohydrate antigens are self-antigens and thus have low antigenic potential. As a consequence, a poor immune response is generated from carbohydrate antigens and the antibody produced is typically a low-affinity immunoglobulin M (IgM).⁷ Conventional hybridoma technologies have been shown to be ineffective at generating high-affinity monoclonal antibodies against a range of carbohydrate structures. In contrast, phage display offers greater potential, as this technology can sometimes generate antibodies against 'self-antigens'.⁸ Moreover, the complementarity-determining regions (CDRs) of scFv fragments can be targeted and mutagenised to enhance their sensitivity and specificity for particular carbohydrate elements.⁹ The vast majority of anti-carbohydrate antibodies generated have relatively low-affinities and are therefore not suitable for *in vitro* diagnostics. To overcome the problem of immunological tolerance, a carefully designed protocol was developed for the generation of a suitable immune response to sialic acid. For our study, *Gallus domesticus* was selected as the animal model. Several reports have suggested that the primary antigen on Neu5Gc, the Hanganutziu-Deicher (HD) antigen, is absent on avian cells. This observation permitted us to select a novel Neu5Gc-containing immunogen for immunisations. We postulate that this sialic acid would be seen as foreign by the avian immune system and thus generate a strong immune response. For T-cell recognition and the subsequent generation of an immune response, the Neu5Gc monosaccharide was conjugated to a suitable carrier protein. Human Serum Albumin (HSA) was deemed to be appropriate as it facilitated the conjugation of multiple Neu5Gc residues. A second sialic acid protein conjugate was also designed. This conjugate, Bovine Serum Albumin (BSA), was used for phage display screening, recombinant protein screening and the measurement of the avian polyclonal serum response.

### 1.1. Synthesis of the Neu5Gc-BSA and Neu5Gc-HSA conjugates

Both the Neu5Gc-BSA and Neu5Gc-HSA conjugates were custom synthesised by Carbohydrate Synthesis, U.K. An overview of the synthesis scheme is given in Figure 1.

### 1.2. Immunisation of chickens using HSA-Neu5Gc

All procedures involving the use of animals were sanctioned by the local ethics committee at Dublin City University (DCU, Dublin, Ireland). In addition, these experiments were approved and licensed by the Irish Department of Health and Children (Dublin, Ireland) and were performed with the highest standards of care. A white male leghorn chicken (aged one month) was injected with 250µg/ml of the HSA-Neu5Gc conjugate (35 monosaccharide units of Neu5Gc per mole of HSA protein) and an equal volume of Freund's complete adjuvant (FCA). The chicken was injected subcutaneously (200µl) at four different sites. Following the first injection, the second, third and fourth boosts were given at two, three, and two weekly intervals respectively. For boosting injections, an equal volume of Freund's incomplete adjuvant was used. A bleed was taken after the fourth boost and a serum-based polyclonal response was determined by ELISA. For comparative analysis, a pre-bleed sample (taken from the same host pre-immunisation) was also selected.

### 1.3. Detection of Sialic acid antibodies in polyclonal avian serum

### 1.3.1. Direct ELISA with BSA-Neu5Gc conjugate

A direct ELISA was used to determine the serum antibody titre from an immunised chicken (Fig. 2a). A Maxisorp plate (Nunc A/S, Denmark) was coated overnight at 4°C with 5µg/ml of the BSA-Neu5Gc conjugate (custom synthesised by Carbohydrate Synthesis, U.K.). The plate was blocked with 3% (w/v) BSA in phosphate-buffered saline (PBS (pH 7.2); NaCl 5.84g/l, Na₂HPO₄ 4.72g/l and NaH₂PO₄ 2.64g/l) for 1 hour at 37°C. The plate was washed three times with PBST, pH 7.2 (PBS containing 0.5% (v/v) Tween) followed by three times with 1x PBS, pH 7.2. A series of dilutions ranging from neat to 1 in 1,000,000 of the chicken serum, diluted in 1% (v/v) BSA 1x PBST (pH 7.2), were added to the ELISA plate in triplicate and incubated for 1 hour at 37°C. The plate was washed three times with 1x PBST (pH 7.2) followed by three times with 1x PBS (pH 7.2). 100µl of rabbit anti-chicken IgY, conjugated with horseradish peroxidase (HRP) (Sigma, U.K., 1:2000 1% (v/v) BSA PBST), was added to the plate and then incubated for 1 hour at 37°C. The plate was washed 3 times with 1x PBST (pH 7.2) and 1x PBS (pH 7.2) and 100µl of TMB substrate (Sigma-Aldrich, U.K.) solution (2mg/ml TMB in citrate 0.05M phosphate-citrate buffer, pH 5.0, Sigma-Aldrich, U.K.) was added to each well. The plate was incubated at room temperature to allow chromophore development, after which the reaction was stopped by the addition of 100µl of 10% (v/v) HCl. The optical density (O.D.) was determined at 450nm with a Tecan Safire plate reader (Tecan, U.K.).

### 1.3.2. Direct ELISA with PAA-Neu5Gc conjugate

To ensure that the avian polyclonal response was directed towards the Neu5Gc component of the conjugate and not the synthetic linker or protein element, the polyclonal serum was also tested against a synthetic carbohydrate that consisted of a multivalent biotinylated polyacrylamide (PAA) polymer that contained 0.2 moles of Neu5Gc per mole of PAA (Glycotech, USA) (Fig. 2b). The serum IgY response against the Neu5Gc antigen was measured by direct ELISA. A 96 well Maxisorp plate was coated overnight at 4°C with 5µg/ml of neutravidin (Pierce, U.K.) prepared in coating buffer (1x PBS, pH 7.2). The plate was washed three times with 1x PBST (pH 7.2) and three times with 1x PBS (pH 7.2). 100µl of biotinylated-PAA-Neu5Gc (25µg/ml) was added to the plate and incubated for one hour at 37°C. The plate was then blocked with 3% (w/v) BSA solution in 1x PBS (pH 7.2) for 1 hour at 37°C. After washing three times with 1x PBST (pH 7.2) and 1x PBS (pH 7.2), 100µl of serially-diluted serum (in 1% (v/v) BSA 1x PBST (pH 7.2) blocking buffer) was added to the relevant wells. After 1 hour at 37°C, the plates were washed as before and 100µl of rabbit anti-chicken IgY conjugated with HRP was added and the plate was then incubated for a further 1 hour. The plate was washed 3 times with 1x PBS (pH 7.2) and 1x PBST (pH 7.2). Subsequently, 100µl of TMB substrate (Sigma-Aldrich, U.K.) solution (2mg/ml TMB in citrate 0.05M phosphate-citrate buffer, pH 5.0, Sigma-Aldrich, U.K.) was added to each well. The plate was incubated at room temperature to allow chromophore development, after which the reaction was stopped by the addition of 100µl of 10% (v/v) HCl. The optical density (O.D.) was determined at 450nm with a Tecan Safire plate reader (Tecan, U.K.).

### 1.3.3. Inhibition ELISA with BSA-Neu5Gc conjugate

A Maxisorp nunc plate was coated overnight at 4°C with 100µl of 5µg/ml of the BSA-Neu5Gc conjugate. The plate was blocked with 3% (w/v) BSA prepared in 1x PBS (pH 7.2) and incubated for 1 hour at 37°C. The plate was washed three times with 1x PBST (pH 7.2) and 1x PBS (pH 7.2). The Neu5Gc-BSA conjugate was added at varying concentrations to a 1:50,000 dilution of avian serum in 1% (v/v) BSA in 1x PBST (pH 7.2). Samples containing no conjugate (A₀) were diluted in 1x PBST (pH 7.2) to ensure the same serum concentration. Sample dilutions were incubated for 2 hours at 37°C and 100µl of sample was added to the relevant wells. After a 1 hour incubation at 37°C, the plates were washed three times with 1x PBST (pH 7.2) and 1x PBS (pH 7.2) and 100µl of rabbit anti-chicken IgY conjugated with HRP was added and the plate was then incubated for 1 hour. The plate was washed three times with 1x PBST (pH 7.2) and 1x PBS (pH 7.2). Subsequently, 100µl of TMB substrate (Sigma-Aldrich, U.K.) solution (2mg/ml TMB in citrate 0.05M phosphate-citrate buffer, pH 5.0, Sigma-Aldrich, U.K.) was added to each well. The plate was incubated at room temperature to allow chromophore development, after which the reaction was stopped by the addition of 100µl of 10% (v/v) HCl. The optical density (O.D.) was determined at 450nm with a Tecan Safire plate reader (Tecan, U.K.).Results are shown in Fig. 2c.

### 2.0. Generation of recombinant anti-sialic acid antibodies

Novel sialic acid binding clones were identified from an avian immune library using the phage display technique. Phage display is a well-established and powerful technique for the discovery and characterisation of antibody fragments (scFv or Fab) that bind to a panel of specific ligands (proteins, carbohydrates or haptens). In this method, antibody fragments are displayed on the outer surface of filamentous phage by inserting short gene fragments in-frame, most commonly into gene III of the phage. This technique couples a polypeptide or peptide of interest to the DNA that encodes it, thus making it possible to select these two characteristics together. The gene III minor coat protein is important for proper phage assembly and for infection by attachment to the pili of *Escherichia coli.* The gene III fusions are translated into chimeric proteins and phage that display proteins with high binding affinities for the target ligand are readily selected. Affinity can be enriched (matured) through multiple rounds of biopanning, a process that involves binding to reducing concentrations of the immobilised ligand. Phage that are weakly bound, or have low affinity for the antigen are removed by stringent washing steps during biopanning. The high-affinity bound phage are removed from the surface of an immunotube/maxisorb plate by acid or trypsin elution, and amplified through infection of mid-logarithmic growth-phase *E. coli* cells. Typically, 4 to 6 rounds of panning and amplification are sufficient to select for phage displaying high-affinity antibody fragments.¹⁰ Many publications exist that describe the display of antibody fragments on the surface of the bacteriophage.¹¹ However, the use of this technique to generate anti-carbohydrate antibody fragments is a much less developed area, although some examples of this have been described.¹² However, no publications currently exist that have used an avian host to generate a recombinant antibody fragment (scFv) that can recognise both major forms of sialic acid (Neu5Gc / Neu5Ac) by phage display.

### 2.1. Isolation and quantification of total cellular RNA from the spleen and bone marrow of an immunised Leghorn chicken.

The immunised Leghorn chicken was sacrificed. Both the spleen and the femurs were immediately harvested and processed in a Laminar flow hood (Gelaire BSB 4) that was thoroughly cleaned with 70% (v/v) industrial methylated spirits (IMS, Lennox) and RNaseZAP^{©} (Invitrogen, USA). The bone marrow from the chicken femurs was washed out with 10 mls of chilled TRIzol^{®} reagent (Invitrogen, USA) using a 25 gauge needle and 5 ml syringe. 10 mls of chilled TRIzol^{®} reagent was added to the avian spleen and all samples were fully homogenised using a sterile (autoclaved and baked overnight at 180°C) homogeniser (Ultra-Turrax model TP 18/10, IKA® Werke GmbH & Co. KG, Germany). The tubes were incubated at room temperature for 5 minutes and centrifuged (eppendorf centrifuge 5810R) at 3500 rpm for 10 minutes at 4°C. The supernatants were carefully removed and transferred to fresh 'RNase-free' 50 ml Oakridge tubes (Thermo Fisher Scientific, USA). For each sample, 3 mls of 'RNase-free' chloroform (Sigma-Aldrich) were added and tubes were shaken vigorously for 15 seconds, stored at room temperature for 15 minutes and subsequently centrifuged at 17,500 rpm at 4°C for an additional 15 minutes. Following centrifugation, the mixture separated into a lower phenolchloroform phase, an interphase, and a colourless upper aqueous phase. The upper aqueous phase, containing the RNA, was carefully removed and transferred to a fresh 'RNase-free' 50 ml Oakridge tube. For each sample, 15mls of propan-2-ol (Sigma-Aldrich) was added and tubes were shaken vigorously for 15 seconds, stored at room temperature for 10 minutes and centrifuged at 17,500 rpm at 4°C for 30 minutes. RNA precipitated as a white gel-like pellet on the bottom and side of the tube. The supernatant was removed and the pellet was washed with 30 mls of 75% (v/v) ethanol (Sigma-Aldrich) and centrifuged at 17,500 rpm at 4°C for 10 minutes. This step was repeated and after removal of the supernatant, the RNA pellet was allowed to air dry for 5 minutes. The pellet was then resuspended in 250µl of 'RNase-free' water (Sigma-Aldrich). The RNA concentrations were determined by spectrophotometric measurement at 260 nm with a NanoDrop^{™} spectrophotometer ND-1000 (Thermo Fisher Scientific, USA). The purity of the RNA preparation was assessed by measuring the ratio of absorbance at 260 nm and 280 nm. Furthermore, sample purity was assessed by analysis on a 1% (w/v) agarose gel. An aliquot of freshly isolated RNA was used for cDNA synthesis. The remaining RNA solution was precipitated at -20°C with 1/10 the volume of 'RNase-free' sodium acetate pH 5.2 (Sigma-Aldrich) and 2 times the total sample volume of 100% (v/v) ethanol. To enhance RNA precipitation, nuclease-free Glycogen (Fermentas) was added at a final concentration of 1µg/ul.

### 2.1.1. Reverse transcription of total RNA to cDNA.

The SuperScript^{™} III First-Strand Synthesis System for RT-PCR, (Invitrogen, USA) was used to generate first strand-cDNA from 5 µg of total RNA using oligo dT₂₀ priming. All reactions were kept on ice at all times. Two 20X master mixes, hereafter referred to as 1 and 2, were made using the recipe below. 25µls of reaction mix 1 were added to 8 tubes that were subsequently incubated at 65°C for 5 minutes (Biometra TGRADIENT PCR machine) and placed on ice for 1 minute. 25µls of reaction mix 2 were added to the same 8 tubes, the tubes were further incubated at 50°C for 50 minutes and the cDNA reaction was terminated by incubation at 85°C for 5 minutes. The samples were spun briefly and 1 µl of RNase H was added to each tube. The tubes were then incubated at 37°C for 20 minutes, after which the cDNA was pooled, aliquoted and stored at -20°C. To assess cDNA quality samples were run on a 1% (w/v) agarose gel.

| Mix 1 - Components | Stock Concentration | Volume per 1 RxN |
|---|---|---|
| RNA | Xµl (to give 5µg) | Xµl (to give 5µg) |
| Oligo-dt | 50µM | 1µl |
| dNTPs | 10mM | 1µl |
| Sterile H₂O | | Xµl |
| Total Volume | | 10µl |
| | | |

| Mix 2 - Components | Stock Concentration | Volume per 1 RxN |
|---|---|---|
| RT Buffer | 10x | 2µl |
| MgCl₂ | 25mM | 4µl |
| DTT | 10mM | 2µl |
| RNase OUT | 40U/µl | 1µl |
| Superscript III RT | 200U/µl | 1µl |
| Total Volume | | 10µl |

### 2.2. PCR Primers and conditions used for the construction of the avian library

The following sets of oligonucleotides were used to generate a chicken scFv library with a short linker from both the bone marrow and spleen. All primers were high purity, salt free and were purchased from Eurofins MWG Operon (Ebersberg, Germany).

### Variable heavy chain (V_{H}) primers

CSCHo-F (sense), Short Linker
   5'GGT CAG TCC TCT AGA TCT TCC GCC GTG AC GTT GGA CGA G 3' (SEQUENCE ID NO: 1)
CSCG-B (reverse)
   5' CTG GCC GGC CTG GCC ACT AGT GGA GGA GAC GAT GAC TTC GGT CC 3' (SEQUENCE ID NO: 2)

### Variable light chain (V_{L)} primers

CSCVK (sense)
   5' GTG GCC CAG GCG GCC CTG ACT CAG CCG TCC TCG GTG TC 3' (SEQUENCE ID NO: 3)
CKJo-B (reverse)
   5' GGA AGA TCT AGA GGA CTG ACC TAG GAC GGT CAG G 3' (SEQUENCE ID NO: 4)

### Overlap extension primers

CSCHo-F (sense)
   5' GAG GAG GAG GAG GAG GAG GTG GCC CAG GCG GCC CTG ACT CAG 3' (SEQUENCE ID NO: 5)
CSC-B (reverse)
   5' GAG GAG GAG GAG GAG GAG GAG CTG GCC GGC CTG GCC ACT AGT GGA GG 3' (SEQUENCE ID NO: 6)

For V_{H} and V_{L} gene amplification, a 100µl PCR reaction contained the following: 1 µl of cDNA, 60 pMole of CSCHo-F and CSCG-B, 5x PCR Buffer (Promega, USA), 1.5mM MgCl₂ (Promega, USA), 200µM dNTPs (Promega, USA), and 0.5µl GoTaq® DNA Polymerase (Promega, USA). For V_{L} gene amplification the PCR reaction components were the same except that 60 pmole of CSCVK and CKJo-B were used in place of the V_{H} primers. The Hybaid Thermal Cycler (Thermo Px2, Thermo Fisher Scientific, USA) was used for all PCR reactions. Touchdown PCR was performed with the following cycling conditions: 4 minutes at 94°C (initial denaturation), followed by 30 cycles of 15 sec at 94°C (denaturation), 30 sec at 60°C (annealing) - the annealing temperature of each cycle was decreased by 0.1 °C, 45 sec at 72°C (extension) and the reaction was terminated after 5 minutes at 72°C (final extension). The resulting PCR products were run analysed a 1% (w/v) agarose gel and purified with the Wizard® SV Gel and PCR Clean-Up System (Promega, USA) according to the manufacturer's instructions. The V_{H} and V_{L} purified fragments were joined with a glycine-serine linker (Gly₄Ser)₃ using splice overlapping extension (SOE) PCR. The resulting PCR product was an amplicon approximately 750 bp in length. For SOE-PCR, a 100µl PCR reaction contained the following: 100ng of the V_{L} and V_{H} purified products, 60 pMolar of CSC-F and CSC-B, 10x PCR Buffer (Invitrogen, USA), 1.5mM MgSO₄ (Invitrogen, USA), 200µM dNTPs and 1µl Platinum® Taq DNA Polymerase (Invitrogen, USA).

PCR was performed with the following cycling conditions: 5 minutes at 94°C (initial denaturation), followed by 30 cycles of 30 sec at 94°C (denaturation), 30 sec at 57°C (annealing), 1 minutes at 72°C (extension) and the reaction was terminated after 10 minutes at 72°C (final extension). The resulting PCR products were run on a 1% (w/v) agarose gel and purified with the Wizard® SV Gel and PCR Clean-Up System according to the manufacturer's instructions.

### 2.2.1. SOE-PCR restriction digestion and ligation into pComb3XSS vector for phage display.

The scFv fragment and the cloning vector pComb3XSS were digestion with the *Sfi* 1 restriction enzyme. Prior to digestion, the vector and scFv DNA concentrations were determined by absorbance measurement at 260nm with the NanoDrop^{™} ND1000 spectrophotometer. For scFv digestion, a 100µl reaction contained the following: 12µg of gel-purified short linker scFv, 200 units of *Sfi* 1 (New England Biolabs, USA), 10x NEBuffer 2 (New England Biolabs, USA) and 10x BSA (New England Biolabs, USA). The pComb3XSS 100µl digestion reaction contained the following: 40µg of gel-purified vector, 240 units of *Sfi1,* 10x NEBuffer 2 and 10x BSA. The digestion of purified insert (scFv) and vector (pComb3XSS) was performed for 5 hours at 50°C.

Following digestion, the cut pComb3XSS vector and the scFv fragment were purified from a 1% (w/v) agarose gel using the Wizard^{®} SV Gel and PCR Clean-Up System and DNA quantification was determined at 260nm using the NanoDrop^{™} ND1000 spectrophotometer. The ligation of the scFv fragment with the pComb3XSS vector (ratio of vector to insert 2:1) was performed using T4 DNA ligase (New England Biolabs, USA) overnight at room temperature. The 200µl ligation mixture contained the following: 1.4µg of gel-purified and stuffer free pComb3XSS vector, 700ng of gel-purified scFv, 5x ligase Buffer, and 200 units of T4 DNA ligase. After ligation, the solution was precipitated at -20°C with 1/10 the volume of 'RNase-free' sodium acetate (pH 5.2), 2 times the volume of 100% (v/v) ethanol and 2µl of pellet paint® NF co-precipitant (Merck, U.K.) After overnight precipitation, the sample was centrifuged at 14000 rpm for 20 minutes at 4°C and the pellet was washed with 70% (v/v) ice-cold ethanol. The mixture was centrifuged at 14000 rpm for 10 minutes at 4°C and the pellet was resuspended in 5µl of molecular grade water (Sigma- Aldrich, USA).

### 2.3. Electro-transformation of XL-1 blue E. coli cells with scFv-containing plasmid.

Commercially available electrocompetent XL-1 blue *E*. *coli* cells (Stratagene, USA) were transformed with the ligated scFv vector construct. This was achieved using a Gene Pulser Xcell electroporation system (Bio-Rad Laboratories, USA) with the controls set at 25µF, 1.25kV and the Pulse Controller at 200Ω. The *E*. *coli* cells (50µl) were thawed on ice. The ligated product (2µl) was added to the cells, mixed, left to incubate for 30 seconds and immediately transferred to an ice-cold 0.2cm electroporation cuvette (Bio-Rad Laboratories, USA). The cuvette was tapped so that the suspension was at the base and was placed in the ShockPod and pulsed once. The cuvette was quickly removed from the chamber and 1 ml of SOC medium (SOB medium containing 20mM glucose; SOB medium contains: tryptone 20g/l, yeast extract 5g/l, NaCl 0.5g/l, 186mg/l KCl, 10mM MgCl₂ and 10mM MgSO₄ (Sigma-Aldrich, USA)) was added immediately to the cuvette. The cells were quickly but gently resuspended with a sterile pasteur pipette. The 1ml suspension was transferred to a 20ml sterile universal container containing 2mls of SOC media. To facilitate recovery of the cells, the universal container was shaken for 1 hour at 250 rpm at 37°C. The pComb3XSS transformants were plated on TYE plates (tryptone 10g/l, yeast extract 5g/l, NaCl 8g/l and bacto agar 15g/l (Sigma-Aldrich, USA)), supplemented with 100 µg/ml carbenicillin (Sigma-Aldrich, USA) and 1 % (v/v) glucose (Sigma-Aldrich, USA). Untransformed XL-1 blue *E*. *coli* cells (negative control) were plated out in parallel on agar plates with 100 µg/ml carbenicillin and 1 % (v/v) glucose. The plates were incubated overnight at 37°C. The pComb3XSS transformant colonies were scraped off the plates and used as library stocks. These cells were suspended in 20 % (v/v) glycerol, snap frozen in liquid nitrogen and stored at -80°C.

### 2.4. Rescue of scfv-displaying phage.

The anti-sialic acid spleen and bone marrow libraries were propagated using 2 x 600µl inoculums of cells (from the frozen glycerol stocks) into 2 x 600mls cultures of 2xTY (tryptone 12g/l; yeast extract 10g/l; NaCl 5g/l; final (pH 7.2) containing 100µg/ml carbenicillin and 2% (w/v) glucose. These libraries were propagated at 200 rpm and 37°C until mid-exponential phase of growth (O.D. ∼ 0.600 @ 600 nm). The cultures were spun down at 4000 rpm at 4°C for 10 minutes. The pellets were resuspended in fresh 2xTY media (600mls) containing 100µg/ml carbenicillin and 1x10¹¹ plaque-forming units (pfu)/ml of M13KO7 helper phage (New England Biolabs, USA). The cultures were incubated at 37°C for 30 minutes without agitation after which time they were propagated at 200 rpm and 37°C for 2 hours. Subsequently, carbenicillin (100µg/ml) and kanamycin (50µg/ml, Sigma-Aldrich, USA) were added and the cultures were grown overnight (200 rpm, 30°C). The cultures were centrifuged at 4000 rpm for 15 minutes at 4°C and the supernatants transferred to clean sterile 250ml Sorval centrifuge tubes (Thermo Fisher Scientific, USA). The phage particles were precipitated by the addition of polyethyleneglycol 8000 (to 4% (w/v)) and NaCl (to 3% (w/v)) (Sigma-Aldrich, USA). The PEG-NaCl solution was dissolved by shaking at 200 rpm for 10 minutes at 37°C. The 250 ml centrifuge tube was placed on ice for 1 hour at 4°C and centrifuged at 8000 rpm for 25 minutes at 4°C. The phage/bacterial pellet was resuspended in 2 ml Tris-EDTA (Sigma-Aldrich, USA) buffer in 2 % (w/v) BSA (Sigma-Aldrich, USA) solution. After the phage pellet was transferred to 1.5 ml sterile centrifuge tubes, it was centrifuged at 14000 rpm for 5 minutes at 4°C. The supernatant containing the phage scFv was placed on ice and stored at 4°C.

### 2.4.1 Selection of sialic acid binding phage scFv fragment by panning with immobilised Neu5Gc-BSA.

Maxisorp immuno-tubes^{tm} (Thermo Fisher Scientific, USA) were coated overnight at 4°C with 500µl of 100µg/ml Neu5Gc-BSA conjugate. The tubes were blocked with 4mls of 3 % (w/v) BSA in 1x PBS (pH 7.2) for 2 hours at room temperature. The blocking solution was removed and 500 µl of rescued phage was added and incubated on a tube roller-mixer SRT1 (Bibby Scientific, U.K.) for 2 hours at room temperature. The solution was removed and non-binding phage were discarded by washing three times with 1x PBST (pH 7.2) and 1x PBS (pH 7.2). Excess PBS was discarded and bound phage particles were eluted with 500µl of 10mg/ml type II porcine trypsin (Sigma-Aldrich) in 1x PBS (pH 7.2) solution. The immuno-tubes^{tm} were then incubated at 37°C for 30 minutes. Half the eluted phage particles (250µl) were stored at 4°C. The other 250µl of phage were infected into 2mls of mid-exponential phase XL-1 blue *E*. *coli* cells. After a static 30 minute incubation at 37°C, 20µl of culture was removed and serial diluted (10⁻¹-10⁻¹²) in 2xTY media. Serial dilutions (10⁻⁸-10⁻⁴) were spread on 2xTY agar plates containing 100µg/ml carbenicillin and incubated overnight at 37°C. The remaining culture was propagated at 200 rpm for 1 hour at 37°C. Cells were harvested by centrifugation at 4000 rpm for 10 minutes at 4°C. Library plates were prepared by resuspending the cell pellet in 600µl of fresh 2xTY media and by spread-plating on TYE plates containing 1% (w/v) glucose and 100µg/ml carbenicillin. Plates were incubated overnight at 37°C.

Input titres were performed by infecting mid-exponential growth phase XL-1 blue *E*. *coli* cells (180µl) with 20µl of precipitated phage (stored at 4°C) for 15 minutes at 37°C and serial dilutions were performed (10⁻¹-10⁻¹²) and spread on 2xTY agar plates containing 100µg/ml carbenicillin and incubated overnight at 37°C. In the subsequent rounds of biopanning (2, 3, 4, and 5) the bone marrow and spleen libraries, only 100mls of 2xTY medium was used for cell propagation. In addition, two different phage elution strategies were followed namely (A) competitive elution and (B) standard tyrpsin elution. For the standard trypsin elution method, the Neu5Gc coating concentrations of the immuno-tubes^{tm} were reduced in rounds 3, 4, and 5 of biopanning to a concentration of 30µg/ml, 20µg/ml, and 10µg/ml, respectively. In addition, the washing of the immuno-tubes^{tm} was increased as follows: round three, 6 times 1x PBST (pH 7.2) and 1x PBS (pH 7.2), round four, 9 times 1x PBST (pH 7.2) and 1x PBS (pH 7.2) and round five, 12 times 1x PBST (pH 7.2) and 1x PBS (pH 7.2). For competitive elution the Neu5Gc-BSA and Neu5Gc-PAA conjugates were added to the immuno-tubes^{tm} and incubated overnight at 4°C.

The next day the eluted phage were infected into 2mls of mid-exponential phase XL-1 blue *E*. *coli* cells. After a static 30 minute incubation at 37°C, 20µl of culture was removed and serial diluted (10⁻¹-10⁻¹²) in 2xTY media. Serial dilutions (10⁻⁸-10⁻⁴) were spread on 2xTY agar plates containing 100µg/ml carbenicillin and incubated overnight at 37°C. The remaining culture was propagated at 200 rpm for 1 hour at 37°C. Cells were harvested by centrifugation at 4000 rpm for 10 minutes at 4°C. Library plates were prepared by resuspending the cell pellet in 600µl of fresh 2xTY media and by spread-plating on TYE plates containing 1% (w/v) glucose and 100µg/ml carbenicillin. Plates were incubated overnight at 37°C. For each successive round of biopanning using competitive elution, 500µl of the Neu5Gc-BSA and Neu5Gc-PAA conjugates in 1% (w/v) BSA 1x PBST (pH 7.2) were added to the immuno-tubes^{tm} at the following concentrations: round 2, 500µg/ml of Neu5Gc-BSA and 40µg/ml Neu5Gc-PAA, round 3, 300µg/ml of Neu5Gc-BSA and 20µg/ml Neu5Gc-PAA, round 4, 200µg/ml of Neu5Gc-BSA and 20µg/ml Neu5Gc-PAA, and round 5, 100µg/ml of Neu5Gc-BSA and 10µg/ml Neu5Gc-PAA. The immuno-tubes^{tm} coating concentration (50µg/ml) and washing (three times 1x PBST (pH 7.2) and 1x PBS (pH 7.2) was kept the same for all rounds of competitive elution panning.

### 2.5. Polyclonal phage ELISA analysis.

For the determination of affinity maturation, a polyclonal phage ELISA was performed. A 96-well plate was coated overnight at 4°C with 100µl of 10µg/ml Neu5Gc-BSA. The plate was then blocked for 1 hour at 37°C with 3% (w/v) BSA in 1x PBS (pH 7.2). After blocking, the plate was washed three times with 1x PBST (pH 7.2) and 1x PBS (pH 7.2). 100µl of phage particles from each round of panning (diluted 1:10 in 1% (w/v) BSA 1x PBST, pH 7.2) were assayed in triplicate. Plates were washed three times with 1x PBST (pH 7.2) and 1x PBS (pH 7.2) and 100µl of a 1:5000 dilution of HRP-conjugated mouse anti-M13 monoclonal antibody (GE Healthcare, U.K.) in 1% (w/v) BSA 1x PBST (pH 7.2) was added for 1 hour at room temperature. The plate was washed three times with 1x PBST (pH 7.2) and 1x PBS (pH 7.2). Subsequently, 100µl of TMB substrate (Sigma-Aldrich, U.K.) solution (2mg/ml TMB in citrate 0.05M phosphate-citrate buffer, pH 5.0, Sigma-Aldrich, U.K.) was added to each well. The plate was incubated at room temperature to allow chromophore development, after which the reaction was stopped by the addition of 100µl of 10% (v/v) HCl. The optical density (O.D.) was determined at 450nm with a Tecan Safire plate reader (Tecan, U.K.).Good affinity maturation for both libraries was seen when assayed on Neu5Gc-BSA.

### 2.6. Production and analysis of soluble antibody fragments.

Antibody fragments without the pIII protein were produced by infecting phagemid DNA from rounds 3 and 4 of panning into *E*. *coli* TOP10F' cells (Stratagene, USA) at mid-logarithmic growth phase. After incubation for 30 minutes at 37°C, serial dilutions were prepared in 2xTY (10⁻² to 10⁻¹⁰), and plated on TYE plates containing 1% (w/v) glucose and 100µg/ml carbenicillin. Single colonies were inoculated into individual wells of a 96-well ELISA plate containing 200µl of 2xTY in the presence of carbenicillin (100µg/ml) and glucose (1.0% (w/v)). After an overnight incubation at 37°C, master plates of the original clones were prepared by adding glycerol (20% (w/v)) and storing at -80°C. These plates were used as a backup stock for each putative clone of interest. Twenty µl from the overnight subculture plates were inoculated into fresh 2xTY media (180µl) containing 1 x 505 medium (0.5 % (v/v) glycerol, 0.05 % (v/v) glucose final concentration), 1mM MgSO₄ and 100µg/ml carbenicillin. The sterile 96 well plates were propagated at 37°C at 180 rpm until a cell density of ∼0.600 was achieved. A final concentration of 1 mM Isopropyl-β-D-thiogalactoside (IPTG) was added to each individual well and the plates were induced overnight at 180 rpm at 30°C. The overnight cultures were frozen at -80°C. The periplasmic scFv was extracted from the cells by three cycles of freeze-thaw. Cell extracts were cleared by centrifugation (4000 rpm, 10 minutes) and the lysates were diluted 1:5 in 1% (w/v) BSA 1x PBS (pH 7.2). ELISA-based analysis was performed as follows. A 96-well plate was coated overnight at 4°C with 100µl of 10µg/ml Neu5Gc-BSA. The plate was then blocked for 1 hour at 37°C with 3% (w/v) BSA in 1x PBS (pH 7.2). After blocking, the plate was washed three times with 1x PBST (pH 7.2) and 1x PBS (pH 7.2). 100µl of the periplasmic scFv cell extracts (diluted 1:5 in 1% (w/v) BSA 1x PBST, pH 7.2) were assayed in triplicate. Plates were washed three times with 1x PBST (pH 7.2) and 1x PBS (pH 7.2) and 100µl of a 1:2000 dilution (1% (w/v) BSA 1x PBST (pH 7.2 ) of rat anti-HA monoclonal antibody conjugated with peroxidase (Roche Diagnostics, USA) was added for 1 hour at 37°C. The plate was washed three times with 1x PBST (pH 7.2) and 1x PBS (pH 7.2). Subsequently, 100µl of TMB substrate (Sigma-Aldrich, U.K.) solution (2mg/ml TMB in citrate 0.05M phosphate-citrate buffer, pH 5.0, Sigma-Aldrich, U.K.) was added to each well. The plate was incubated at room temperature to allow chromophore development, after which the reaction was stopped by the addition of 100µl of 10% (v/v) HCl. The optical density (O.D.) was determined at 450nm with a Tecan Safire plate reader (Tecan, U.K.). Results for competitively-eluted and trypsin eluted scFvs are shown in Figs. 3a and 3b, respectively.

### 2.7. Assessing the ability of the soluble anti-sialic clones to recognise Neu5Gc in the context of a polyacrylamide backbone.

In order to identify scFvs that could not only bind Neu5Gc-BSA but also recognise this monosaccharide in the context of an alternative backbone, positive clones identified from monoclonal scFv ELISA-analysis, were tested against Neu5Gc-PAA. A 96 well nunc maxisorb plate was coated overnight at 4°C with 5µg/ml of neutravidin in coating buffer 1x PBS (pH 7.2). The plate was washed three times with 1x PBS (pH 7.2) and 1x PBST (pH 7.2). 100µl of biotinylated-PAA-Neu5Gc (25µg/ml) was added and the plate was incubated for one hour at 37°C. The plate was blocked with 3% (w/v) BSA solution in 1x PBS (pH 7.2) for 1 hour at 37°C. After washing three times with 1x PBS (pH 7.2) and 1x PBST (pH 7.2), 100µl of soluble-expressed scFv (diluted 1:5 in 1% (w/v) BSA 1x PBST, pH 7.2) were added to the plate. After a 1 hour incubation at 37°C, the plate was washed three times with 1x PBS (pH 7.2) and 1x PBST (pH 7.2) and 100µl of 1:2000 dilution (1% (w/v) BSA 1x PBST (pH 7.2)) of an anti-HA rat monoclonal antibody conjugated with peroxidase was added. The plate was incubated for 1 hour, washed 3 times with 1x PBS (pH 7.2) and 1x PBST (pH 7.2). Subsequently, 100µl of TMB substrate (Sigma-Aldrich, U.K.) solution (2mg/ml TMB in citrate 0.05M phosphate-citrate buffer, pH 5.0, Sigma-Aldrich, U.K.) was added to each well. The plate was incubated at room temperature to allow chromophore development, after which the reaction was stopped by the addition of 100µl of 10% (v/v) HCl. The optical density (O.D.) was determined at 450nm with a Tecan Safire plate reader (Tecan, U.K.). Results for this assay are illustrated in Fig. 3c.

### 2.7.1. Cross reaction-analysis of the soluble anti-Neu5Gc clones with other mono and disaccharides.

The capacity of the anti-Neu5Gc clones to cross-react with other carbohydrate elements was assessed by analysis against the following structures: Neu5Ac-PAA, (Neu5Ac)₂-PAA, Neu5Gc-DOPE, glucose-PAA and galactose-PAA (Fig 4). A 96 well nunc maxisorb plate was coated overnight at 4°C with 5µg/ml of neutravidin in coating buffer 1x PBS (pH 7.2). The plate was washed with three times with 1x PBS (pH 7.2) and 1x PBST (pH 7.2). 100µl of biotinylated-PAA-conjugate (Neu5Ac-PAA, (Neu5Ac)₂-PAA, Neu5Gc-DOPE, glucose-PAA and galactose-PAA) at 25µg/ml was added and the plate was incubated for one hour at 37°C. The plate was blocked with 3% (w/v) BSA solution in 1x PBS (pH 7.2) for 1 hour at 37°C. After washing three times with 1x PBS (pH 7.2) and 1x PBST (pH 7.2), 100µl of soluble-expressed scFv (diluted 1:5 in 1% (w/v) BSA 1x PBST, pH 7.2) were added to the plate. After a 1 hour incubation at 37°C, the plate was washed three times with 1x PBS (pH 7.2) and 1x PBST (pH 7.2) and 100µl of 1:2000 dilution (1% (w/v) BSA 1x PBST (pH 7.2)) of an anti-HA rat monoclonal antibody conjugated with peroxidase was added. The plate was incubated for 1 hour, washed 3 times with 1x PBS (pH 7.2) and 1x PBST (pH 7.2). Subsequently, 100µl of TMB substrate (Sigma-Aldrich, U.K.) solution (2mg/ml TMB in citrate 0.05M phosphate-citrate buffer, pH 5.0, Sigma-Aldrich, U.K.) was added to each well. The plate was incubated at room temperature to allow chromophore development, after which the reaction was stopped by the addition of 100µl of 10% (v/v) HCl. The optical density (O.D.) was determined at 450nm with a Tecan Safire plate reader (Tecan, U.K.).

### 2.8. Sequence analysis of the anti-Neu5Gc / Neu5Ac binding clones

To ensure the fidelity of the sequence data, three different samples (stab culture, plasmid prep and unpurified PCR products) of the same clone were sent for sequencing. Double stranded DNA sequencing of all clones was performed by Eurofins MWG Operon (Ebersberg, Germany). A panel of anti-sialic acid clones were grown in 1.5ml eppendorf stab cultures. In addition, purified plasmid was obtained from each clone using the Wizard® Plus SV Minipreps DNA Purification System in accordance with the manufacturer's instructions. Furthermore, the scFv gene fragment was also amplified using colony pick PCR. For colony pick PCR, a 50µl PCR reaction contained the following: 2µl of an overnight culture, 60 pmole of CSC-F and CSC-B, 5x PCR Buffer, 1.5mM MgCl₂, 200µM dNTPs and 0.25µl GoTaq® DNA Polymerase. Touchdown PCR was performed with the following cycling conditions: 10 minutes at 94°C (initial denaturation), followed by 30 cycles of 30 sec at 94°C (denaturation), 30 sec at 56°C (annealing) - the annealing temperature of each cycle was decreased by 0.1 °C, 1 minute at 72°C (extension) and the reaction was terminated after 10 minutes at 72°C (final extension). The sequences of the CDLR1, CDLR2, CDLR3, CDHR1, CDHR2 and CDHR3 regions for the AE8, AG9, CD3, and CC1 clones are shown in Fig. 5. The full sequences of the variable heavy and light chains of the clones, and the sequences of the full clones (including the spacer which is underlined) are provided below:

### AE8

**Variable Light Chain** 
GGTVKITCSGGGGSYYGWFQQKSPGSAPVTVIYDNTNRPSNIPSRFSGSLSGSTNTLTITG
VQAEDEAVYYCGSYDRSAGYVGIFGAGTTLTVL (SEQUENCE ID NO: 22)
**Variable Heavy Chain** 
AVTLDESGGGLQTPGGGLSLVCKASGFTFDSYAMYWVRQAPGKGLEWVASINRFGSST
GHGAAVKGRATISRDNGQSTLGAYPYDVPDYAS (SEQUENCE ID NO: 23)
**scFv**

GGTVKITCSGGGGSYYGWFQQKSPGSAPVTVIYDNTNRPSNIPSRFSGSLSGSTNTLTITG
VQAEDEAVYYCGSYDRSAGYVGIFGAGTTLTVLGQSSRSSAVTLDESGGGLQTPGGGLS
LVCKASGFTFDSYAMYWVRQAPGKGLEWVASINRFGSSTGHGAAVKGRATISRDNGQS
TLGAYPYDVPDYAS (SEQUENCE ID NO: 30)

### AE9

**Variable Light Chain** 
GGTVKITCSGGGGSYYGWFQQKSPGSAPVTVIYDNTNRPSNIPSRFSGSKSGSTGTLTITV
QAEDEAVYYCGNFDTSAIFGAGTTLTVL (SEQUENCE ID NO: 24)
**Variable Heavy Chain** 
AVTLDESGGGLQTPGGALSLICKASGFTFSSFNMIWVRQAPGKGLEFVGSINRFGNSTGH
GAAVKGRVTISRDDGQSTVRLQLNNLRAEDTGTYFCAKSVIIGIICASGYWCSAASIDA
WGHGTEVIVSSTSGQAGQHHHHHH GAYPYDVPDYAS (SEQUENCE ID NO: 25)
**scFv**

GGTVKITCSGGGGSYYGWFQQKSPGSAPVTVIYDNTNRPSNIPSRFSGSKSGSTGTLTITV
QAEDEAVYYCGNFDTSAIFGAGTTLTVLGQSSRSSAVTLDESGGGLQTPGGALSLICKAS
GFTFSSFNMIWVRQAPGKGLEFVGSINRFGNSTGHGAAVKGRVTISRDDGQSTVRLQLN
NLRAEDTGTYFCAKSVHGHCASGYWCSAASIDAWGHGTEVIVSSTSGQAGQHHHHHH
GAYPYDVPDYAS (SEQUENCE ID NO: 31)

### CD3

**Variable Light Chain** 
GGTVEITCSGGSYSYGWYQQKSPGSAPVTVIYQNTNRPSDIPSRFSGSKSGSTGTLTITGV
RAEDEAVYYCGSFDSSVGMFGAGTTLTVL (SEQUENCE ID NO: 26)
**Variable Heavy Chain** 
AVTLDESEGGLQTPGGALSLVCKASGFSFSDRGMHWVRQAPGKGLEYVAGIYDDGGTT
YYGAAVKGRASITRDNGQSAVRLQLNNLRAEDTATYYCAKSAAGDAWGADDIDAWG
HGTEVIVSSTSGQAGQHHHHHHGAYPYDVPDYAS (SEQUENCE ID NO: 27)
**scFv**

GGTVEITCSGGSYSYGWYQQKSPGSAPVTVIYQNTNRPSDIPSRFSGSKSGSTGTLTITGV
RAEDEAVYYCGSFDSSVGMFGAGTTLTVLGQSSRSSAVTLDESEGGLQTPGGALSLVCK
ASGFSFSDRGMHWVRQAPGKGLEYVAGIYDDGGTTYYGAAVKGRASITRDNGQSAVR
LQLNNLRAEDTATYYCAKSAAGDAWGADDIDAWGHGTEVIVSSTSGQAGQHHHHHH
GAYPYDVPDYAS (SEQUENCE ID NO: 32)

### CC11

**Variable Light Chain** 
KWYGWYQQKAPGSAPVTLIYDNTNRPSDIPSRFSGSASGSTATLTITGVQVEDEAVYFG
GYDGSTDAGIFGAGTTLTVL (SEQUENCE ID NO: 28)
**Variable heavy Chain** 
AVTLDESGGGLQTPGGALSLVCKASGFDFSSYQMNWIRQAPGKGLEWVAAINKFGTST
SRGAAVKGRVTISRDDGQSTVRLQLNNLRSEDTATYFCAKSAYGSCASGSWCSAASIDA
WGHGTEVIVSSTSGQAGQHHHHHHGAYPYDVPDYAS (SEQUENCE ID NO: 29)
**scFv**

KWYGWYQQKAPGSAPVTLIYDNTNRPSDIPSRFSGSASGSTATLTITGVQVEDEAVYFC
GGYDGSTDAGIFGAGTTLTVLGQSSRSSAVTLDESGGGLQTPGGALSLVCKASGFDFSS
YQMNWIRQAPGKGLEWVAAINKFGTSTSRGAAVKGRVTISRDDGQSTVRLQLNNLRSE
DTATYFCAKSAYGSCASGSWCSAASIDAWGHGTEVIVSSTSGQAGQHHHHHHGAYPY
DVPDYAS (SEQUENCE ID NO: 33)

### 2.9. Immobilised metal affinity chromatography (IMAC) purification

For further analysis by HPLC and Surface Plasmon Resonance (SPR), the AE8 clone was purified by immobilised metal affinity chromatography (IMAC). A single colony of the AE8 clone was sub-cultured into 5mls of 2xTY containing 100µg/ml carbenicillin and 1% (w/v) glucose and grown overnight at 37°C. Five hundred µls of the overnight culture was inoculated into 500mls of terrific-broth (TB; tryptone 13.3g/l, yeast extract 26.6g/l and glycerol 0.44%, (v/v)) that contained 1x 505 medium (0.5 % (v/v) glycerol, 0.05 % (v/v) glucose final concentration), 50mls potassium phosphate solution (KH₂PO₄ 2.31g/1, K₂HPO₄ 12.54g/l), 1mM MgSO₄ and 100µg/ml carbenicillin. The culture was incubated at 37°C at 240 rpm until an approximate OD₆₀₀ of 0.6 was reached. The culture was then induced with 1mM IPTG and incubated at 30°C overnight at 240 rpm. The following day, the culture was centrifuged at 4000 rpm for 10 minutes at 4°C and the pellet was completely re-suspended in 30mls of ice-cold sonication buffer (1x PBS, 0.5M NaCl and 20mM imidazole) and then aliquoted (1ml) into 1.5ml Eppendorf tubes. Each individual sample was sonicated on ice for 45 seconds (40% amplitude) with 6 sec pulses for 3 minutes. The samples were then centrifuged at 14,000 rpm for 10 minutes at 4°C. The lysates were pooled, filtered through a 0.2µM filter and stored at 4°C. All IMAC purifications were performed using PD-10 columns (GE Healthcare, U.K.). Two millilitres of Ni-NTA resin (Qiagen, USA) were added to the column and allowed to form a packed bed. After equilibration with 30mls of running buffer (sonication buffer containing 1% (v/v) Tween), the pooled lysate was then added to the column and the flow-through was collected and stored at 4°C. The column was subsequently washed with 30mls of running buffer and the bound scFv was eluted by adding 20mls of 100mM sodium acetate (pH 4.4). 400µl volumes of eluent were added to 50µl of 10x PBS (pH 7.2) and 50µl of 100mM NaOH before mixing. Individual fractions were tested for the presence of protein by quantification at 280nm with the Nanodrop ND1000 spectrophotometer. Those fractions that contained the eluted scFv were pooled and concentrated using a 5000Da molecular weight cut-off (MWCO) buffer exchange column (Sartorius, Germany). The scFv-containing sample was concentrated to a volume of 500µl by centrifugation (4000 rpm) at 4°C. Five mls of 1x PBS were subsequently added to the column and, after an overnight incubation at 4°C, the sample was buffer exchanged and re-concentrated by centrifugation until the final volume was approximately 200µl. Protein concentration was determined by quantification at 280nm using a Nanodrop ND1000 spectrophotometer (Labtech International, U.K.).

### 2.10. SEC-HPLC analysis of scFv clone AE8

HPLC size exclusion chromatography (SEC-HPLC) was used to determine the species composition, apparent molecular weight and to purify the monomeric fraction of the recombinant AE8 scFv. A Shimadzu LC system (Shimadzu Corporation, Japan), equipped with a Shimadzu CBM-20A controller, Shimadzu LC-20AB pumps, Shimadzu SPD-20A UV-Vis spectrophotometric detector, Shimadzu SIL-20A autosampler, Shimadzu FRC-10A fraction collector, Shimadzu CTO-20AC column oven and Shimadzu's LCsolution software for data handling. The experiments were carried out using the size exclusion Bio-Sep-SEC-S2000 column (Phenomenex; 300x7.8mm) protected with a guard column (Phenomenex; 35x7.8mm). The HPLC system was operated isocratically at room temperature using filtered and degassed 1x PBS (pH 7.2) as the mobile phase. Prior to sample analysis, the column was equilibrated for 45 minutes by gradually increasing the flow rate in increments of 0.1 ml/minutes. All samples (20µl) were diluted in 1x PBS (pH 7.2) and assayed at a flow rate of 0.5mls/minutes with UV detection (280nm). The following protein standards (Agilent, USA) were used: bovine thyroglobulin (670kD), Human gamma globulin (IgG; 150kD), ovalbumin (44kD) and myoglobin (17kD). Samples were interspersed with water blanks to ensure that all residual protein was eluted. The monomeric AE8 scFv was isolated with the Bio-Sep-SEC-S2000 HPLC column by the collection of several fractions between 17.4 and 18.2minutes at a flow rate of 0.5ml/minute (Fig. 6).

### 2.11. FPLC analysis of scFv clone AE8

Fast protein liquid chromatography (FPLC) was used to estimate the molecular weight of the AE8 protein. The ÄKTA^{™} Explorer 100 system (GE Healthcare, USA) equipped with a UV-900 monitor, monitor pH/C-900, sample pump, fraction collector frac-950 and UNICORN™ software for data handling was used for protein analysis. 100µl of the AE8 sample was applied to a HiLoad™ 16/60 Superdex™ 200 Prep-grade FPLC column using filtered and degassed 1x PBS (pH 7.2) at a flow rate of 1ml/min. The following protein standards (Agilent, USA): bovine thyroglobulin (670kD), Human gamma globulin (IgG; 150kD), ovalbumin (44kD) and myoglobin (17kD) were used for the molecular weight estimation of the scFv (Fig 7).

### 3.0 Surface plasmon resonance analysis of the AE8 clone using the BIAcore^{®} 3000 biosensor.

Analysis of the binding and kinetic properties of the AE8 clone was performed using the BIAcore^{®} 3000 biosensor which monitors 'label-free' biomolecular interactions in 'real-time' using the phenomenon of surface plasmon resonance (SPR). The basic assay format for AE8 SPR analysis was as follows: neutravidin was immobilised on the dextran surface of a BIAcore^{®} CM5 chip, biotinylated polyacrylamide Neu5Gc conjugate was then passed over and captured by the neutravidin, after which the scFv was passed over the surface to check for binding to the sugar. As a negative control, the scFv was also passed over a neutravidin surface which had no biotinylated sugar.

### 3.1 Pre-concentration, immobilisation of neutravidin on a carboxy-methylated dextran chip and capture of biotinylated-Neu5Gc polyacrylamide (PAA).

For all BlAcore^{®} 3000 (GE Healthcare, Sweden) experiments, the running buffer used was filtered and degassed HEPES buffered saline pH 7.4 (HBS: 50mM NaCl, 10mM HEPES, 3.4mM EDTA and 0.05% (v/v) Tween-20). 50µg/ml solutions of neutravidin (Thermo Fisher Scientific, USA) were prepared in 10mM sodium acetate (Sigma-Aldrich, USA) buffers that had been adjusted with 10% (v/v) acetic acid (Sigma-Aldrich, USA) to pH values 4.0, 4.2, 4.4, 4.6, 4.8, and 5.0. 20µl of protein at each respective pH was sequentially passed over the underivatised carboxy-methylated dextran sensor chip surface (CM5, GE Healthcare, Sweden) at a flow-rate of 10µl/minute. A pH of 4.6 was determined to be the optimal pH for neutravidin immobilisation as this yielded the largest change in response units (RU). Neutravidin was immobilised on the CM5 chip with the following protocol: 70µl of 400mM of 1-ethyl-3-[3-dimethylaminopropyl] carbodiimide hydrochloride (EDC) (GE Healthcare, Sweden ) was mixed with 70µl of 100mM N-hydroxysuccinimide (NHS) (GE Healthcare, Sweden ) and injected over the sensor chip surface for 10 minutes at a flowrate of 10µl/minute. A 50µg/ml solution of neutravidin was prepared in 10mM sodium acetate (Sigma-Aldrich, USA), pH 4.6 and injected over the activated chip surface for 24 minutes at a flow-rate of 10µl/minute. Unreacted NHS ester groups were capped and loose, non-covalently attached proteins were removed by injection of 1M ethanolamine hydrochloride (GE Healthcare, Sweden ), pH 8.5, for 11 minutes. Four 30 second sequential pulses of 5mM NaOH at a flow-rate of 10µl/minute were used to remove any other loosely bound material. After neutravidin immobilisation (Fig 8b), a 100µg/ml solution of biotinylated-Neu5Gc-PAA in HBS was passed over the chip surface at 10µl/min for 20 minutes (Fig 8c).

### 3.2. SPR analysis of the AE8 clone.

The sialic acid binding ability of the AE8 clone was assessed with the previously prepared Neu5Gc sensor chip. A 1 in 100 dilution of the IMAC purified AE8 clone in HBS was simultaneously passed over flow cells 1 and 2 of the sensor chip at a flow rate of 10µl/min for 7 minutes. Following on-line reference subtraction (2-1), the sensorgram indicated a response increase of 1,077.4 RU above baseline. Bound antibody was dissociated with a 30 second pulse of 10mM NaOH and the baseline was restored with the injection of HBS running buffer over the chip surface. The experiment was repeated five times and, on each occasion, the AE8 Neu5Gc binding response was greater than 1000 RU.

### 3.3 Solution-phase Neu5Gc-binding assay

To assess the ability of the anti-sialic acid scFv to bind the sialic acid conjugate in solution-phase, an inhibition binding assay was performed. The purified AE8 scFv was diluted 1 in 2000 in HBS buffer (pH 7.4). The Neu5Gc-BSA conjugate was also diluted in HBS buffer (pH 7.4) to the following concentrations: 2000ng/ml, 1000ng/ml, 500ng/ml, 250ng/ml, 125ng/ml and 62.5ng/ml. 100µl of the AE8 sample was mixed with 100µl of each of the Neu5Gc-BSA conjugate dilutions to yield the following free conjugate working concentrations: 1000ng/ml, 500ng/ml, 250ng/ml, 125ng/ml, 62.5ng/ml and 31.25ng/ml. The zero conjugate sample contained 100µl of 1 in 2000 dilution of the purified AE8 scFv in HBS buffer (pH 7.4) and 100µl of HBS buffer (pH 7.4). Samples were incubated for 1 hour at 37°C and then injected (40µl), in random order, over flow cells 1 and 2 of the Neu5Gc chip at a flow rate of 10µl/minute for 4 minutes and the change in response recorded. Bound antibody was removed by injection of 5µl of 5mM NaOH at a flow rate of 10µl/minute for 30 seconds. The amount of free antigen necessary to cause 50% displacement of antibody (IC₅₀) was 5.7ng/ml.

### 3.4 Preliminary SPR kinetic studies on the AE8 clone

SPR was used to determine the association and dissociation rate constants of the anti-sialic acid scFv. The rate constants were fitted with a pre-defined fitting algorithm using the Biaevaluation 4.1 software. To avoid mass-transfer limited binding, a smaller quantity (1µg/ml) of neutravidin (<10,000 RU) was immobilised (see section 3.1) on the sensor chip surface. Subsequently, for the capture step, a 40ng/ml solution of biotinylated-Neu5Gc-PAA in HBS buffer (pH 7.4) was passed over the chip surface at 10µl/min for 1 minute. A final level of 28.6 RU of captured biotinylated-Neu5Gc-PAA was achieved. Furthermore, to rule out the contribution of avidity in the determination of the rate constants, only the monomeric HPLC-purified fraction of AE8 was used for Biacore kinetic analysis. The rate constants were calculated using different concentrations (6.67µg/ml, 4.44µg/ml, 2.96µg/ml, 1.98µg/ml, 1.32µg/ml, 0.88µg/ml, 0.59µg/ml and 0µg/ml) of monomeric scFv diluted in HBS buffer (pH 7.4). The kinject command was used to inject 90µl of each sample over flow cells 1 and 2 of the Neu5Gc sensor chip, at a flow rate of 30µl/minute for 3 minutes with a dissociation time of 12 minutes. The zero scFv sample was analysed twice and all samples were run in random order. To reflect the PBS composition of the HPLC eluted monomeric scFv, the zero scFv sample contained 1x PBS (pH 7.2) diluted 1 in 10 in HBS buffer (pH 7.4). Bound antibody was removed by injection of 5µl of 1.25mM NaOH at a flow rate of 30µl/minute for 10 seconds. All sensorgrams were reference-subtracted from flow cell 1, which contained a blank dextran surface. In addition, to remove systematic anomalies a blank run consisting of a zero concentration of the scFv samples was subtracted from each of the sensorgrams.

### References

1. Kelm, S., and Schauer, R. (1997). Sialic acids in molecular and cellular interactions. Int. Rev. Cytol. 175:37-240.
2. Schauer, R. (2000). Achievements and challenges of sialic acid research. Glycoconjugate J. 17:495-499.
3. Varki, A. (2008). Sialic acids in human health and disease. Trends Mol. Med. 14:(8)351-60.
4. Varki, A., Freeze, H., H., Manzi, A., E. (2001). Overview of glycoconjugate analysis. Curr. Protoc. Protein Sci. 12:12.1.
5. Varki, N., M., and Varki, A. (2007). Diversity in cell surface sialic acid presentations: implications for biology and disease. Lab Invest. 87(9):851-7.
6. Angata, T. and Varki, A. (2002). Chemical diversity in the sialic acids and related α-keto acids: an evolutionary perspective. Chem. Rev. 102:439-469.
7. Slovin, F., S., Keding, J., S., Ragupathi., G. (2005). Carbohydrate vaccines as immunotherapy for cancer. Immunol. Cell Biol. 83:418-428.
8. Sakai, K., Shimizu, Y., Chiba, T., Matsumoto-Takasaki, A., Kusada, Y., Zhang, W., Nakata, M., Kojima, N., Toma, K., Takayanagi, A., Shimizu, N., Yoko Fujita-Yamaguchi, Y. (2007). Isolation and Characterization of Phage-Displayed Single Chain Antibodies Recognizing Nonreducing Terminal Mannose Residues. 1. A New Strategy for Generation of Anti-Carbohydrate Antibodies. Biochemistry 46(1):253-262.
9. Deng, S., Roger M., C., Sadowska, J., Michniewicz, J., Martin, Y., N., Bundle, R., D., Narang, A., S. (1994). Selection of Antibody Single-chain Variable Fragments with Improved Carbohydrate Binding by Phage Display. J. Biological Chemistry 269(13):9533-38.
10. Smith, G., P. (1985). Filamentous fusion phage: novel expression vectors that display cloned antigens on the virion surface. Science 228(4705):1315-7.
11. Vaughan, T., J., Osbourn, J., K., Tempest, P., R. (1998). Human antibodies by design. Nature Biotechnology 16(6):535-9.
12. Schoonbroodt, S., Steukers, M., Viswanathan, M., Frans, N., Timmermans, M., Wehnert, A., Nguyen, M., Ladner, R., C., Hoet, R., M. (2008). Engineering antibody heavy chain CDR3 to create a phage display Fab library rich in antibodies that bind charged carbohydrates. J Immunol. 181(9):6213-21.

## Claims

1. A method of generating and isolating recombinant high affinity anti-sialic acid antibody molecules, the method comprising the steps of: immunising an avian host with an immunogen comprising a conjugate of sialic acid and a carrier protein to generate an anti-sialic acid polyclonal serum; isolating a sample of RNA from the immunised avian host; using phage display in the generation and screening of a library of recombinant avian antibody molecules from the RNA sample; and isolating recombinant high affinity anti-sialic acid antibody molecules.

2. A method as claimed in Claim 1 in which the carrier protein is a serum albumin protein, and the sialic acid is either Neu5Gc or Neu5Ac.

3. A method as claimed in Claim 1 or 2 in which the the sialic acid is conjugated to the carrier protein by a linker comprising a hydrocarbon chain having at least seven carbon atoms.

4. A method as claimed in any preceding Claim in which the antibody molecule is selected from the group consisting of: whole antibodies; scFv fragments; and Fab fragments.

5. A method as claimed in any preceding Claim in which the avian host is *Gallus domesticus.*

6. A recombinant avian antibody fragment having high binding affinity to sialic acid and obtainable by the method of any of Claims 1 to 5.

7. An anti-sialic acid polyclonal serum obtainable by immunising an avian host with a conjugate of sialic acid and carrier protein.

8. A recombinant avian anti-sialic acid antibody molecule having a nanomolar binding affinity to sialic acid.

9. A recombinant avian anti-sialic acid antibody molecule according to Claim 8, the antibody molecule comprising a light chain variable region having a CDRL1 region according to SEQUENCE ID NO: 12, 13 or 14, a CDRL2 region according to SEQUENCE ID NO: 7 or 8, and a CDRL3 region according to SEQUENCE ID NO'S: 9, 10 or 11, and a heavy chain variable region having a CDRH1 region according to SEQUENCE ID NO: 15, 16 or 17, a CDRH2 region according to SEQUENCE ID NO: 18 or 19, and a CDRH3 region according to SEQUENCE ID NO'S: 20 or 21, or a functional variant of the antibody molecule.

10. A recombinant avian anti-sialic acid antibody molecule according to Claim 9 in which the light chain variable region comprises a CDRL1 region according to SEQUENCE ID NO: 14, a CDRL2 region according to SEQUENCE ID NO: 8, a CDRL3 region according to SEQUENCE ID NO'S: 11, and the heavy chain variable region comprises a CDRH1 region according to SEQUENCE ID NO: 17, a CDRH2 region according to SEQUENCE ID NO: 19, and a CDRH3 region according to SEQUENCE ID NO'S: 21, or a functional variant of the antibody molecule.

11. A recombinant avian anti-sialic acid antibody molecule according to Claim 8 in which the light chain variable region comprises a sequence according to SEQUENCE ID NO: 22, or a functional variant thereof, and the heavy chain variable region comprising a sequence according to SEQUENCE ID NO: 23, or a functional variant thereof.

12. A recombinant avian anti-sialic acid antibody molecule according to Claim 8, in which the light chain variable region comprises a sequence according to SEQUENCE ID NO: 24, or a functional variant thereof, and the heavy chain variable region comprising a sequence according to SEQUENCE ID NO: 25, or a functional variant thereof.

13. A recombinant avian anti-sialic acid antibody molecule according to Claim 8, in which the light chain variable region comprises a sequence according to SEQUENCE ID NO: 26, or a functional variant thereof, and the heavy chain variable region comprises a sequence according to SEQUENCE ID NO: 27, or a functional variant thereof.

14. A recombinant avian anti-sialic acid antibody molecule according to Claim 8, in which the light chain variable region comprises a sequence according to SEQUENCE ID NO: 28, or a functional variant thereof, and the heavy chain variable region comprises a sequence according to SEQUENCE ID NO: 29, or a functional variant thereof.

15. A recombinant avian anti-sialic acid antibody molecule according to Claim 8 comprising a sequence selected from the group consisting of: SEQUENCE ID NO: 30; 31; 32; and 33.


SEQUENCE LISTING

<110>  DUBLIN CITY UNIVERSITY

<120>  ANTI-SIALIC ACID ANTIBODY MOLECULES

<130>  10335pr/BP

<160>  33

<170>  PatentIn version 3.3

<210>  1
<211>  39
<212>  DNA
<213>  Artificial

<220>
<223>  Variable Heavy Chain Forward primer

<400>  1
ggtcagtcct ctagatcttc cgccgtgacg ttggacgag                              39


<210>  2
<211>  44
<212>  DNA
<213>  Artificial

<220>
<223>  Variable heavy Chain Reverse Primer

<400>  2
ctggccggcc tggccactag tggaggagac gatgacttcg gtcc                        44


<210>  3
<211>  38
<212>  DNA
<213>  Artificial

<220>
<223>  Variable Ligh Chain Forward primer

<400>  3
gtggcccagg cggccctgac tcagccgtcc tcggtgtc                               38


<210>  4
<211>  34
<212>  DNA
<213>  Artificial

<220>
<223>  Variable Light Chain Reverse Primer

<400>  4
ggaagatcta gaggactgac ctaggacggt cagg                                   34


<210>  5
<211>  42
<212>  DNA
<213>  Artificial

<220>
<223>  Overlap Extension Forward Primer

<400>  5
gaggaggagg aggaggaggt ggcccaggcg gccctgactc ag                          42


<210>  6
<211>  47
<212>  DNA
<213>  Artificial

<220>
<223>  Overlap Extension Reverse Primer

<400>  6
gaggaggagg aggaggagga gctggccggc ctggccacta gtggagg                     47


<210>  7
<211>  7
<212>  PRT
<213>  Artificial

<220>
<223>  CDRL2 region


<220>
<221>  MISC_FEATURE
<222>  (1)..(1)
<223>  X is any amino acid

<400>  7

Xaa Asn Thr Asn Arg Pro Ser
1               5


<210>  8
<211>  7
<212>  PRT
<213>  Artificial

<220>
<223>  CDRL2 region

<400>  8

Asp Asn Thr Asn Arg Pro Ser
1               5


<210>  9
<211>  12
<212>  PRT
<213>  Artificial

<220>
<223>  CDRL3 region


<220>
<221>  MISC_FEATURE
<222>  (2)..(2)
<223>  X is any amino acid

<220>
<221>  MISC_FEATURE
<222>  (3)..(3)
<223>  X is Y or F

<220>
<221>  MISC_FEATURE
<222>  (5)..(5)
<223>  X is any amino acid

<220>
<221>  MISC_FEATURE
<222>  (7)..(12)
<223>  X is any amino acid

<400>  9

Gly Xaa Xaa Asp Xaa Ser Xaa Xaa Xaa Xaa Xaa Xaa
1               5                   10


<210>  10
<211>  12
<212>  PRT
<213>  Artificial

<220>
<223>  CDRL3 region


<220>
<221>  misc_feature
<222>  (2)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (8)..(11)
<223>  Xaa can be any naturally occurring amino acid

<400>  10

Gly Xaa Xaa Asp Xaa Ser Ala Xaa Xaa Xaa Xaa Ile
1               5                   10


<210>  11
<211>  12
<212>  PRT
<213>  Artificial

<220>
<223>  CDRL3 region

<400>  11

Gly Ser Tyr Asp Arg Ser Ala Gly Tyr Val Gly Ile
1               5                   10


<210>  12
<211>  9
<212>  PRT
<213>  Artificial

<220>
<223>  CDRL1 region

<220>
<221>  MISC_FEATURE
<222>  (1)..(7)
<223>  X is any amino acid

<400>  12

Xaa Xaa Xaa Xaa Xaa Xaa Xaa Tyr Gly
1               5


<210>  13
<211>  9
<212>  PRT
<213>  Artificial

<220>
<223>  CDRL1 region


<220>
<221>  MISC_FEATURE
<222>  (4)..(5)
<223>  X is any amino acid

<220>
<221>  MISC_FEATURE
<222>  (7)..(7)
<223>  X is any amino acid

<400>  13

Ser Gly Gly Xaa Xaa Ser Xaa Tyr Gly
1               5


<210>  14
<211>  9
<212>  PRT
<213>  Artificial

<220>
<223>  CDRL1 region

<400>  14

Ser Gly Gly Gly Gly Ser Tyr Tyr Gly
1               5


<210>  15
<211>  10
<212>  PRT
<213>  Artificial

<220>
<223>  CDRH1 region


<220>
<221>  MISC_FEATURE
<222>  (3)..(3)
<223>  X is any amino acid

<220>
<221>  MISC_FEATURE

<222>  (5)..(8)
<223>  X is any amino acid

<220>
<221>  MISC_FEATURE
<222>  (10)..(10)
<223>  X is any amino acid

<400>  15

Gly Phe Xaa Phe Xaa Xaa Xaa Xaa Met Xaa
1               5                   10


<210>  16
<211>  10
<212>  PRT
<213>  Artificial

<220>
<223>  CDRH1 region


<220>
<221>  MISC_FEATURE
<222>  (5)..(5)
<223>  X is any amino acid

<220>
<221>  MISC_FEATURE
<222>  (7)..(8)
<223>  X is any amino acid

<220>
<221>  MISC_FEATURE
<222>  (10)..(10)
<223>  X is any amino acid

<400>  16

Gly Phe Thr Phe Xaa Ser Xaa Xaa Met Xaa
1               5                   10


<210>  17
<211>  10
<212>  PRT
<213>  Artificial

<220>
<223>  CDRH1 region

<400>  17

Gly Phe Thr Phe Asp Ser Tyr Ala Met Tyr
1               5                   10


<210>  18
<211>  14
<212>  PRT
<213>  Artificial

<220>
<223>  CDRH2 region


<220>

<221>  MISC_FEATURE
<222>  (2)..(4)
<223>  X is any amino acid

<220>
<221>  MISC_FEATURE
<222>  (6)..(7)
<223>  X is any amino acid

<220>
<221>  MISC_FEATURE
<222>  (9)..(10)
<223>  X is any amino acid

<400>  18

Ile Xaa Xaa Xaa Gly Xaa Xaa Thr Xaa Xaa Gly Ala Ala Val
1               5                   10


<210>  19
<211>  14
<212>  PRT
<213>  Artificial

<220>
<223>  CDRH2 region


<220>
<221>  MISC_FEATURE
<222>  (6)..(6)
<223>  X is S or N

<400>  19

Ile Asn Arg Phe Gly Xaa Ser Thr Gly His Gly Ala Ala Val
1               5                   10


<210>  20
<211>  19
<212>  PRT
<213>  Artificial

<220>
<223>  CDRH3 region


<220>
<221>  MISC_FEATURE
<222>  (2)..(3)
<223>  X is any amino acid

<220>
<221>  MISC_FEATURE
<222>  (5)..(16)
<223>  X is any amino acid

<400>  20

Ser Xaa Xaa Gly Xaa Xaa Xaa Xaa Xaa Xaa Xaa Xaa Xaa Xaa Xaa Xaa
1               5                   10                  15


Ile Asp Ala

<210>  21
<211>  19
<212>  PRT
<213>  Artificial

<220>
<223>  CDRH3 region


<220>
<221>  MISC_FEATURE
<222>  (5)..(5)
<223>  X is S or H

<220>
<221>  MISC_FEATURE
<222>  (10)..(10)
<223>  X is T or Y

<220>
<221>  MISC_FEATURE
<222>  (14)..(14)
<223>  X is P or A

<400>  21

Ser Val His Gly Xaa Cys Ala Ser Gly Xaa Trp Cys Ser Xaa Ala Ser
1               5                   10                  15


Ile Asp Ala




<210>  22
<211>  94
<212>  PRT
<213>  Artificial

<220>
<223>  Variable Light Chain - Clone AE8

<400>  22

Gly Gly Thr Val Lys Ile Thr Cys Ser Gly Gly Gly Gly Ser Tyr Tyr
1               5                   10                  15


Gly Trp Phe Gln Gln Lys Ser Pro Gly Ser Ala Pro Val Thr Val Ile
            20                  25                  30


Tyr Asp Asn Thr Asn Arg Pro Ser Asn Ile Pro Ser Arg Phe Ser Gly
        35                  40                  45


Ser Leu Ser Gly Ser Thr Asn Thr Leu Thr Ile Thr Gly Val Gln Ala
    50                  55                  60


Glu Asp Glu Ala Val Tyr Tyr Cys Gly Ser Tyr Asp Arg Ser Ala Gly
65                  70                  75                  80


Tyr Val Gly Ile Phe Gly Ala Gly Thr Thr Leu Thr Val Leu
                85                  90

<210>  23
<211>  91
<212>  PRT
<213>  Artificial

<220>
<223>  Variable Heavy Chain - AE8

<400>  23

Ala Val Thr Leu Asp Glu Ser Gly Gly Gly Leu Gln Thr Pro Gly Gly
1               5                   10                  15

Gly Leu Ser Leu Val Cys Lys Ala Ser Gly Phe Thr Phe Asp Ser Tyr
            20                  25                  30

Ala Met Tyr Trp Val Arg Gln Ala Pro Gly Lys Gly Leu Glu Trp Val
        35                  40                  45

Ala Ser Ile Asn Arg Phe Gly Ser Ser Thr Gly His Gly Ala Ala Val
    50                  55                  60

Lys Gly Arg Ala Thr Ile Ser Arg Asp Asn Gly Gln Ser Thr Leu Gly
65                  70                  75                  80

Ala Tyr Pro Tyr Asp Val Pro Asp Tyr Ala Ser
                85                  90


<210>  24
<211>  89
<212>  PRT
<213>  Artificial

<220>
<223>  Variable Light Chain - Clone AE9

<400>  24

Gly Gly Thr Val Lys Ile Thr Cys Ser Gly Gly Gly Gly Ser Tyr Tyr
1               5                   10                  15

Gly Trp Phe Gln Gln Lys Ser Pro Gly Ser Ala Pro Val Thr Val Ile
            20                  25                  30

Tyr Asp Asn Thr Asn Arg Pro Ser Asn Ile Pro Ser Arg Phe Ser Gly
        35                  40                  45

Ser Lys Ser Gly Ser Thr Gly Thr Leu Thr Ile Thr Val Gln Ala Glu
    50                  55                  60

Asp Glu Ala Val Tyr Tyr Cys Gly Asn Phe Asp Thr Ser Ala Ile Phe
65                  70                  75                  80

Gly Ala Gly Thr Thr Leu Thr Val Leu
                85

<210>  25
<211>  153
<212>  PRT
<213>  Artificial

<220>
<223>  Variable Heavy Chain - AE9

<400>  25

Ala Val Thr Leu Asp Glu Ser Gly Gly Gly Leu Gln Thr Pro Gly Gly
1               5                   10                  15

Ala Leu Ser Leu Ile Cys Lys Ala Ser Gly Phe Thr Phe Ser Ser Phe
            20                  25                  30

Asn Met Ile Trp Val Arg Gln Ala Pro Gly Lys Gly Leu Glu Phe Val
        35                  40                  45

Gly Ser Ile Asn Arg Phe Gly Asn Ser Thr Gly His Gly Ala Ala Val
    50                  55                  60

Lys Gly Arg Val Thr Ile Ser Arg Asp Asp Gly Gln Ser Thr Val Arg
65                  70                  75                  80

Leu Gln Leu Asn Asn Leu Arg Ala Glu Asp Thr Gly Thr Tyr Phe Cys
                85                  90                  95

Ala Lys Ser Val His Gly His Cys Ala Ser Gly Tyr Trp Cys Ser Ala
            100                 105                 110

Ala Ser Ile Asp Ala Trp Gly His Gly Thr Glu Val Ile Val Ser Ser
        115                 120                 125

Thr Ser Gly Gln Ala Gly Gln His His His His His His Gly Ala Tyr
    130                 135                 140

Pro Tyr Asp Val Pro Asp Tyr Ala Ser
145                 150


<210>  26
<211>  90
<212>  PRT
<213>  Artificial

<220>
<223>  Variable Light Chain - CD3

<400>  26

Gly Gly Thr Val Glu Ile Thr Cys Ser Gly Gly Ser Tyr Ser Tyr Gly
1               5                   10                  15

Trp Tyr Gln Gln Lys Ser Pro Gly Ser Ala Pro Val Thr Val Ile Tyr
            20                  25                  30

Gln Asn Thr Asn Arg Pro Ser Asp Ile Pro Ser Arg Phe Ser Gly Ser
        35                  40                  45

Lys Ser Gly Ser Thr Gly Thr Leu Thr Ile Thr Gly Val Arg Ala Glu
    50                  55                  60

Asp Glu Ala Val Tyr Tyr Cys Gly Ser Phe Asp Ser Ser Val Gly Met
65                  70                  75                  80

Phe Gly Ala Gly Thr Thr Leu Thr Val Leu
                85                  90

<210>  27
<211>  148
<212>  PRT
<213>  Artificial

<220>
<223>  Variable Heavy Chain - Clone CD3

<400>  27

Ala Val Thr Leu Asp Glu Ser Glu Gly Gly Leu Gln Thr Pro Gly Gly
1               5                   10                  15

Ala Leu Ser Leu Val Cys Lys Ala Ser Gly Phe Ser Phe Ser Asp Arg
            20                  25                  30

Gly Met His Trp Val Arg Gln Ala Pro Gly Lys Gly Leu Glu Tyr Val
        35                  40                  45

Ala Gly Ile Tyr Asp Asp Gly Gly Thr Thr Tyr Tyr Gly Ala Ala Val
    50                  55                  60

Lys Gly Arg Ala Ser Ile Thr Arg Asp Asn Gly Gln Ser Ala Val Arg
65                  70                  75                  80

Leu Gln Leu Asn Asn Leu Arg Ala Glu Asp Thr Ala Thr Tyr Tyr Cys
                85                  90                  95

Ala Lys Ser Ala Ala Gly Asp Ala Trp Gly Ala Asp Asp Ile Asp Ala
            100                 105                 110

Trp Gly His Gly Thr Glu Val Ile Val Ser Ser Thr Ser Gly Gln Ala
        115                 120                 125

Gly Gln His His His His His His Gly Ala Tyr Pro Tyr Asp Val Pro
    130                 135                 140

Asp Tyr Ala Ser
145

<210>  28

<211>  79
<212>  PRT
<213>  Artificial

<220>
<223>  Variable Light Chain - Clone CD11

<400>  28

Lys Trp Tyr Gly Trp Tyr Gln Gln Lys Ala Pro Gly Ser Ala Pro Val
1               5                   10                  15

Thr Leu Ile Tyr Asp Asn Thr Asn Arg Pro Ser Asp Ile Pro Ser Arg
            20                  25                  30

Phe Ser Gly Ser Ala Ser Gly Ser Thr Ala Thr Leu Thr Ile Thr Gly
        35                  40                  45

Val Gln Val Glu Asp Glu Ala Val Tyr Phe Gly Gly Tyr Asp Gly Ser
    50                  55                  60

Thr Asp Ala Gly Ile Phe Gly Ala Gly Thr Thr Leu Thr Val Leu
65                  70                  75

<210>  29
<211>  153
<212>  PRT
<213>  Artificial

<220>
<223>  Variable Heavy Chain - Clone CC11

<400>  29

Ala Val Thr Leu Asp Glu Ser Gly Gly Gly Leu Gln Thr Pro Gly Gly
1               5                   10                  15

Ala Leu Ser Leu Val Cys Lys Ala Ser Gly Phe Asp Phe Ser Ser Tyr
            20                  25                  30

Gln Met Asn Trp Ile Arg Gln Ala Pro Gly Lys Gly Leu Glu Trp Val
        35                  40                  45

Ala Ala Ile Asn Lys Phe Gly Thr Ser Thr Ser Arg Gly Ala Ala Val
    50                  55                  60

Lys Gly Arg Val Thr Ile Ser Arg Asp Asp Gly Gln Ser Thr Val Arg
65                  70                  75                  80

Leu Gln Leu Asn Asn Leu Arg Ser Glu Asp Thr Ala Thr Tyr Phe Cys
                85                  90                  95

Ala Lys Ser Ala Tyr Gly Ser Cys Ala Ser Gly Ser Trp Cys Ser Ala
            100                 105                 110

Ala Ser Ile Asp Ala Trp Gly His Gly Thr Glu Val Ile Val Ser Ser

115                 120                 125

Thr Ser Gly Gln Ala Gly Gln His His His His His His Gly Ala Tyr
    130                 135                 140

Pro Tyr Asp Val Pro Asp Tyr Ala Ser
145                 150

<210>  30
<211>  192
<212>  PRT
<213>  Artificial

<220>
<223>  Clone  AE8 scFv

<400>  30

Gly Gly Thr Val Lys Ile Thr Cys Ser Gly Gly Gly Gly Ser Tyr Tyr
1               5                   10                  15

Gly Trp Phe Gln Gln Lys Ser Pro Gly Ser Ala Pro Val Thr Val Ile
            20                  25                  30

Tyr Asp Asn Thr Asn Arg Pro Ser Asn Ile Pro Ser Arg Phe Ser Gly
        35                  40                  45

Ser Leu Ser Gly Ser Thr Asn Thr Leu Thr Ile Thr Gly Val Gln Ala
    50                  55                  60

Glu Asp Glu Ala Val Tyr Tyr Cys Gly Ser Tyr Asp Arg Ser Ala Gly
65                  70                  75                  80

Tyr Val Gly Ile Phe Gly Ala Gly Thr Thr Leu Thr Val Leu Gly Gln
                85                  90                  95

Ser Ser Arg Ser Ser Ala Val Thr Leu Asp Glu Ser Gly Gly Gly Leu
            100                 105                 110

Gln Thr Pro Gly Gly Gly Leu Ser Leu Val Cys Lys Ala Ser Gly Phe
        115                 120                 125

Thr Phe Asp Ser Tyr Ala Met Tyr Trp Val Arg Gln Ala Pro Gly Lys
    130                 135                 140

Gly Leu Glu Trp Val Ala Ser Ile Asn Arg Phe Gly Ser Ser Thr Gly
145                 150                 155                 160

His Gly Ala Ala Val Lys Gly Arg Ala Thr Ile Ser Arg Asp Asn Gly
                165                 170                 175

Gln Ser Thr Leu Gly Ala Tyr Pro Tyr Asp Val Pro Asp Tyr Ala Ser
            180                 185                 190

<210>  31
<211>  249
<212>  PRT
<213>  Artificial

<220>
<223>  Clone AE9 scFv

<400>  31

Gly Gly Thr Val Lys Ile Thr Cys Ser Gly Gly Gly Gly Ser Tyr Tyr
1               5                   10                  15

Gly Trp Phe Gln Gln Lys Ser Pro Gly Ser Ala Pro Val Thr Val Ile
            20                  25                  30

Tyr Asp Asn Thr Asn Arg Pro Ser Asn Ile Pro Ser Arg Phe Ser Gly
        35                  40                  45

Ser Lys Ser Gly Ser Thr Gly Thr Leu Thr Ile Thr Val Gln Ala Glu
    50                  55                  60

Asp Glu Ala Val Tyr Tyr Cys Gly Asn Phe Asp Thr Ser Ala Ile Phe
65                  70                  75                  80

Gly Ala Gly Thr Thr Leu Thr Val Leu Gly Gln Ser Ser Arg Ser Ser
                85                  90                  95

Ala Val Thr Leu Asp Glu Ser Gly Gly Gly Leu Gln Thr Pro Gly Gly
            100                 105                 110

Ala Leu Ser Leu Ile Cys Lys Ala Ser Gly Phe Thr Phe Ser Ser Phe
        115                 120                 125

Asn Met Ile Trp Val Arg Gln Ala Pro Gly Lys Gly Leu Glu Phe Val
    130                 135                 140

Gly Ser Ile Asn Arg Phe Gly Asn Ser Thr Gly His Gly Ala Ala Val
145                 150                 155                 160

Lys Gly Arg Val Thr Ile Ser Arg Asp Asp Gly Gln Ser Thr Val Arg
                165                 170                 175

Leu Gln Leu Asn Asn Leu Arg Ala Glu Asp Thr Gly Thr Tyr Phe Cys
            180                 185                 190

Ala Lys Ser Val His Gly His Cys Ala Ser Gly Tyr Trp Cys Ser Ala
        195                 200                 205

Ala Ser Ile Asp Ala Trp Gly His Gly Thr Glu Val Ile Val Ser Ser
    210                 215                 220

Thr Ser Gly Gln Ala Gly Gln His His His His His His Gly Ala Tyr

225                 230                 235                 240

Pro Tyr Asp Val Pro Asp Tyr Ala Ser
                245

<210>  32
<211>  245
<212>  PRT
<213>  Artificial

<220>
<223>  Clone CD3 scFv

<400>  32

Gly Gly Thr Val Glu Ile Thr Cys Ser Gly Gly Ser Tyr Ser Tyr Gly
1               5                   10                  15

Trp Tyr Gln Gln Lys Ser Pro Gly Ser Ala Pro Val Thr Val Ile Tyr
            20                  25                  30

Gln Asn Thr Asn Arg Pro Ser Asp Ile Pro Ser Arg Phe Ser Gly Ser
        35                  40                  45

Lys Ser Gly Ser Thr Gly Thr Leu Thr Ile Thr Gly Val Arg Ala Glu
    50                  55                  60

Asp Glu Ala Val Tyr Tyr Cys Gly Ser Phe Asp Ser Ser Val Gly Met
65                  70                  75                  80

Phe Gly Ala Gly Thr Thr Leu Thr Val Leu Gly Gln Ser Ser Arg Ser
                85                  90                  95

Ser Ala Val Thr Leu Asp Glu Ser Glu Gly Gly Leu Gln Thr Pro Gly
            100                 105                 110

Gly Ala Leu Ser Leu Val Cys Lys Ala Ser Gly Phe Ser Phe Ser Asp
        115                 120                 125

Arg Gly Met His Trp Val Arg Gln Ala Pro Gly Lys Gly Leu Glu Tyr
    130                 135                 140

Val Ala Gly Ile Tyr Asp Asp Gly Gly Thr Thr Tyr Tyr Gly Ala Ala
145                 150                 155                 160

Val Lys Gly Arg Ala Ser Ile Thr Arg Asp Asn Gly Gln Ser Ala Val
                165                 170                 175

Arg Leu Gln Leu Asn Asn Leu Arg Ala Glu Asp Thr Ala Thr Tyr Tyr
            180                 185                 190

Cys Ala Lys Ser Ala Ala Gly Asp Ala Trp Gly Ala Asp Asp Ile Asp
        195                 200                 205

Ala Trp Gly His Gly Thr Glu Val Ile Val Ser Ser Thr Ser Gly Gln
    210                 215                 220

Ala Gly Gln His His His His His His Gly Ala Tyr Pro Tyr Asp Val
225                 230                 235                 240

Pro Asp Tyr Ala Ser
                245

<210>  33
<211>  240
<212>  PRT
<213>  Artificial

<220>
<223>  Clone CD11 scFv

<400>  33

Lys Trp Tyr Gly Trp Tyr Gln Gln Lys Ala Pro Gly Ser Ala Pro Val
1               5                   10                  15

Thr Leu Ile Tyr Asp Asn Thr Asn Arg Pro Ser Asp Ile Pro Ser Arg
            20                  25                  30

Phe Ser Gly Ser Ala Ser Gly Ser Thr Ala Thr Leu Thr Ile Thr Gly
        35                  40                  45

Val Gln Val Glu Asp Glu Ala Val Tyr Phe Cys Gly Gly Tyr Asp Gly
    50                  55                  60

Ser Thr Asp Ala Gly Ile Phe Gly Ala Gly Thr Thr Leu Thr Val Leu
65                  70                  75                  80

Gly Gln Ser Ser Arg Ser Ser Ala Val Thr Leu Asp Glu Ser Gly Gly
                85                  90                  95

Gly Leu Gln Thr Pro Gly Gly Ala Leu Ser Leu Val Cys Lys Ala Ser
            100                 105                 110

Gly Phe Asp Phe Ser Ser Tyr Gln Met Asn Trp Ile Arg Gln Ala Pro
        115                 120                 125

Gly Lys Gly Leu Glu Trp Val Ala Ala Ile Asn Lys Phe Gly Thr Ser
    130                 135                 140

Thr Ser Arg Gly Ala Ala Val Lys Gly Arg Val Thr Ile Ser Arg Asp
145                 150                 155                 160

Asp Gly Gln Ser Thr Val Arg Leu Gln Leu Asn Asn Leu Arg Ser Glu
                165                 170                 175

Asp Thr Ala Thr Tyr Phe Cys Ala Lys Ser Ala Tyr Gly Ser Cys Ala

180                 185                 190

Ser Gly Ser Trp Cys Ser Ala Ala Ser Ile Asp Ala Trp Gly His Gly
        195                 200                 205

Thr Glu Val Ile Val Ser Ser Thr Ser Gly Gln Ala Gly Gln His His
    210                 215                 220

His His His His Gly Ala Tyr Pro Tyr Asp Val Pro Asp Tyr Ala Ser
225                 230                 235                 240